# EUROPEAN PATENT SPECIFICATION

(11) **EP 0 958 367 B1**
(45) Date of publication and mention of the grant of the patent: **08.06.2005**
(21) Application number: 97915941.5
(22) Date of filing: 03.03.1997
(51) Int. Cl.: C12N 15/52, C12N 15/54, C12N 15/70, C12N 1/21, C12P 7/62, C12N 15/82, C12N 5/14

(54) **METHODS FOR THE BIOSYNTHESIS OF POLYESTERS**
VERFAHREN ZUR POLYESTERN-BIOSYNTHESE
METHODES POUR REALISER LA BIOSYNTHESE DE POLYESTERS

(43) Date of publication of application: 24.11.1999
(73) Proprietor: Metabolix, Inc., Cambridge, MA 02139 (US)
(72) Inventor: HEIN, Silke, D-48143 Münster (DE); SÖHLING, Brigitte, D-06110 Halle (DE); GOTTSCHALK, Gerhard, D-37176 Nörten-Hardenberg (DE); STEINBÜCHEL, Alexander, D-48341 Altenberge (DE)
(74) Representative: Bassett, Richard Simon
(86) International application number: PCT/US1997/003994
(87) International publication number: WO 1998/039453

(56) References cited:
- WO-A-93/06225
- WO-A-95/21257
- US-A- 5 518 907
- CHEMICAL ABSTRACTS, vol. 127, Columbus, Ohio, US; abstract no. 219607, HEIN, SILKE ET AL: "Biosynthesis of poly(4-hydroxybutyric acid) by recombinant strains of Escherichia coli" XP002047459 & FEMS MICROBIOL. LETT. (1997), 153(2), 411-418 CODEN: FMLED7;ISSN: 0378-1097,

## Description

### FIELD OF THE INVENTION

The invention relates generally to the field of molecular biology. Certain embodiments entail materials and methods suitable for the biosynthesis of biopolymers, namely polyhydroxyalkanoic acid polymers.

### BACKGROUND OF THE INVENTION

The production of intracellular polyesters belonging to the class of polymers known as polyhydroxyalkanoates (polyhydroxyalkanoic acids) has been observed in a wide array of prokaryotic organisms (Anderson. A.J. and Dawes, E.A. (1990) *Microbiol. Rev.* 54:450-472; Steinbüchel, A. and Valentin. H.E. (1995) *FEMS Microbiol. Lett*. 128:219-228). The monomers composing the polyesters range in length from C4 (3-hydroxybutyrate) to C12 (3-hydroxydodecanoate) (Lageveen, R.G. et al. (1988) *Appl. Env. Microbiol*. 54:2924-2932). These polyesters have attracted considerable interest as they are biodegradable. Potential technical applications exist in industry and agriculture, as well as in medical devices and procedures (Hocking, P.J. and Marchessault. R.H. (1994) Biopolyesters. In: G.J.L. Griffin (Ed) Chemistry and technology of biodegradable polymers, Chapman & Hall, London, pp.48-96; Müller, H.M. and Seebach, D. (1993) *Angew. Chem*. 105:483-509). Additionally, this class of polyesters is attractive as a potential alternative to conventional petrochemical-derived plastics.

Polyhydroxyalkanoic acids are broadly characterized according to the monomers that constitute their backbone. Polymers composed of C4-C5 units are classified as short chain length (scl) polyhydroxyalkanoic acids; polymers containing monomers of C6 units and above are classified as medium chain length (mcl) polyhydroxyalkanoic acids. The primary structure of the polymer influences the physical properties of the polyester.

The metabolic pathways leading to the formation of polyhydroxyalkanoic acids have not been elucidated for all organisms. The most extensively studied polyhydroxyalkanoic acid biosynthetic pathway is that of *Alcaligenes* (Peoples, O.P. et al. (1989) *J. Biol. Chem.* 264:15298-15303; Valentin, H.E. et al. (1995) *Eur. J. Biochem.* 227:43-60). This organism is capable of forming either a homopolymer of C4 (polyhydroxybutyrate, PHB) or a co-polymer of C4-C5 (PHB-PHV, polyhydroxybutyrate-polyhydroxyvalerate) (Koyama, N. and Doi, Y. (1995) *Biotechnol. Lett.* 17:281-284). Hence, *A. eutrophus* is classified as a scl polyhydroxyalkanoic acid organism. Similarly, *Pseudomonas* species generate a polymer composed of monomers ranging in length from C6 to C12 (Timm, A. and Steinbüchel, A. (1990) *Appl. Environ. Microbiol.* 56:3360; Lageveen, R.G. et al. (1988) *Appl. Env. Microbiol.* 54:2924-2932), and are classified as mcl polyhydroxyalkanoic acid organisms.

The polymerization of the hydroxyacyl-CoA substrates is carried out by polyhydroxyalkanoic acid synthases. The substrate specificity of this class of enzyme varies across the spectrum of polyhydroxyalkanoic acid producing organisms. This variation in substrate specificity of polyhydroxyalkanoic acid synthases is supported by indirect evidence observed in heterologous expression studies (Lee, E.Y. et al. (1995) *Appl. Microbiol. Biotechnol*. 42:901-909; Timm, A. et al. (1990) *Appl. Microbiol. Biotechnol*. 33:296-301). Hence, the structure of the backbone of the polymer is strongly influenced by the polyhydroxyalkanoic acid synthase responsible for its formation.

Fluorescent pseudomonads belonging to the rRNA homology group I can synthesize and accumulate large amounts of polyhydroxyalkanoic acids (PHA) composed of various saturated and unsaturated hydroxy fatty acids with carbon chain lengths ranging from 6 to 14 carbon atoms (Steinbüchel, A. and Valentin. H.E. (1992) *FEMS Microbiol. Rev.* 103:217). Polyhydroxyalkanoic acid isolated from these bacteria also contains constituents with functional groups such as branched, halogenated, aromatic or nitrile side-chains (Steinbüchel and Valentin (1995 *FEMS Microbiol. Lett*. 128:219-228). The composition of polyhydroxyalkanoic acid depends on the polyhydroxyalkanoic acid polymerase system, the carbon source, and the metabolic routes (Anderson, A.J. and Dawes, E.A. (1990) *Microbiol. Rev.* 54:450-472; Eggink et al. (1992) *FEMS Microbiol. Rev.* 105:759; Huisman, A.M. et al. (1989) *Appl. Microbiol. Biotechnol*. 55:1949-1954; Lenz, O. et al. (1992) *J. Bacteriol.* 176:4385-4393; Steinbüchel, A. and Valentin, H.E. (1995) *FEMS Microbiol. Lett.* 128:219-228). In *P. putida*, at least three different metabolic routes occur for the synthesis of 3-hydroxyacyl CoA thioesters, which are the substrates of the polyhydroxyalkanoic acid synthase (Huijberts, G.N.M. et al. (1994) *J. Bacteriol.* 176:1661-1666): (i) β-oxidation is the main pathway when fatty acids are used as carbon source; (ii) *De novo* fatty acid biosynthesis is the main route during growth on carbon sources which are metabolized to acetyl-CoA, like gluconate, acetate or ethanol; and (iii) Chain elongation reaction, in which acyl-CoA is condensed with acetyl-CoA to the two carbon chain extended β-keto product which is then reduced to 3-hydroxyacyl-CoA. This latter pathway is involved in polyhydroxyalkanoic acid-synthesis during growth on hexanoate.

The polyhydroxyalkanoic acid synthase structural gene from *Alcaligenes eutrophus* (*phaC*_{*Ae*}) has been cloned and characterized at the molecular level in several laboratories (for a review see Steinbüchel, A. and Schlegel, H.G. (1991) *Mol. Microbiol*. 5:53 5-542; GenBank Accession number J05003). It was demonstrated that *phaC*_{*Ae*} in combination with other genes conferred the ability to synthesize poly(3-hydroxybutyric acid) not only to many bacteria, which do not synthesize this polyester such as e.g. *Escherichia coli* (Steinbüchel, A. and Schlegel, H.G. (1991) *Mol. Microbiol*. 5:535-542) but also to *Saccharomyces cerevisiae* (Leaf, T.A. et al. (1996) *Microbiology* 142:1169-1180), plants such as *Arabidopsis thaliana* (Poirier, Y. et al. (1992) *Science* 256:520-523.) and *Gossypium hirsutum* (John, M.E. and Keller, G. (1996) *Proc. Natl. Acad. Sci. U.S.A*. 93:12768-12773), and even to cells from the insect *Spodoptera frugiperda* (Williams, M.D. et al. (1996) *Appl. Environ. Microbiol*. 62:2540-2546).

The development of biological systems that synthesize poly (4-hydroxybutyric acid), poly (3-hydroxybutyric acid -co- 4-hydroxybutyric acid), and other polyester materials would be of great utility. Biological systems provide the potential to produce significant quantities of important materials, while utilizing inexpensive feedstocks and minimizing hazardous byproducts.

There exists a need for novel biosynthetic routes to polymers of potential commercial interest that do not rely on petroleum based starting materials. Biological processes present an attractive alternative to chemical processes that produce potentially harmful byproducts while consuming non-renewable resources.

### SUMMARY

This invention relates to materials and processes for preparing polyester materials. More particularly, the invention is related to materials and processes for the preparation of polyester materials, preferably poly (4-hydroxybutyric acid), poly (3-hydroxybutyric acid), and poly (3-hydroxybutyric acid -co- 4-hydroxybutyric acid). The invention provides nucleic acid segments encoding a recombinant polyhydroxyalkanoic acid synthase protein and a recombinant fatty acid:acyl-CoA transferase protein, or an acyl-CoA synthetase protein, recombinant vectors, cells containing the nucleic acid segments, and methods for the preparation of polyester materials.

The scope of the present invention will be further apparent in light of the detailed descriptions provided below. However, it should be understood that the following detailed description and examples, while indicating preferred embodiments of the present invention, are given by way of illustration only since various changes and modifications within the spirit and scope of the present invention will become apparent to those of ordinary skill in the art from this detailed description.

### Definitions

The following definitions are provided in order to aid those skilled in the art in understanding the detailed description of the present invention.

An "acyl-CoA synthetase" or "thiokinase" protein catalyzes the formation of a thioester linkage between the carboxyl group of a fatty acid and the sulfhydryl group of CoA.

An "acyl kinase" protein catalyzes the transfer of a phosphate group from ATP to a carboxylate group according to the reaction: 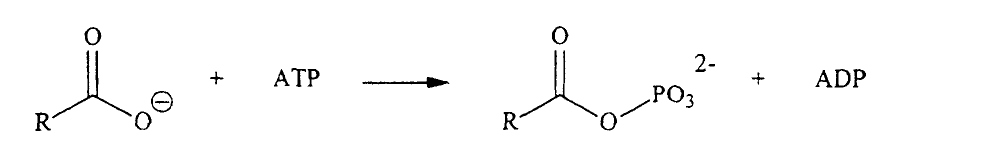 wherein R is an alkyl or hydroxyalkyl group.

"CoA" refers to coenzyme A.

The term "fatty acid:acyl-Co A transferase" refers to a protein that catalyzes an acyl group transfer according to the reaction: 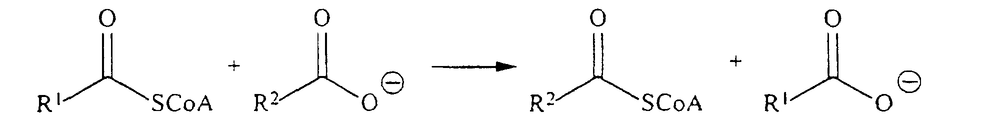 wherein R¹ and R² are alkyl or hydroxyalkyl groups. Groups R¹ and R² may further contain one or more double bonds, triple bonds, or aromatic groups.

"Copolyester" refers to a polyester material made from a two different monomeric building blocks. For example, poly (3-hydroxybutyric acid -co- 4-hydroxybutyric acid) is a polyester made from 3-hydroxybutyric acid and 4-hydroxybutyric acid. The relative composition of the two monomeric building blocks in the copolyester can be variable. Copolyesters are commonly characterized by the relative percentages of the two monomeric building blocks. The percentage composition may affect the physical characteristics of the copolyester.

"Dimeric" refers to enzymes that are comprised of two protein molecule subunits. The two subunits may be identical (homodimeric) or different (heterodimeric) in sequence.

"Heterologous" refers to nucleotide segments not normally found in nature in the same organism.

"Homopolyester" refers to a polyester material made from a single monomeric building block. For example, poly (4-hydroxybutyric acid) is a polyester made from 4-hydroxybutyric acid.

A "4-hydroxybutyrate dehydrogenase" protein catalyzes the conversion of succinate semialdehyde to 4-hydroxybutyrate.

The combination of "2-methylcitrate dehydratase" protein and "2-methylisocitrate dehydratase" protein catalyzes the conversion of 2-methylcitrate to 2-methylisocitrate.

A "2-methylcitrate synthase" protein catalyzes the conversion of propionyl-CoA and oxaloacetate to 2-methylcitrate.

A "2-methylisocitrate lyase" protein catalyzes the conversion of 2-methylisocitrate to pyruvate and succinate.

The terms "microbe", "microorganism", and "microbial" refer to algae, bacteria, fungi, and protozoa.

"Monomeric" refers to enzymes that are comprised of a single protein molecule.

"Native" refers to two segments of nucleic acid naturally occurring in the same organism. For example, a native promoter is the promoter naturally found with a given gene in an organism.

"Nucleic acid" refers to deoxyribonucleic acid (DNA) and ribonucleic acid (RNA).

"Overexpression" refers to the expression of a polypeptide or protein encoded by a DNA introduced into a host cell, wherein said polypeptide or protein is either not normally present in the host cell, or wherein said polypeptide or protein is present in said host cell at a higher level than that normally expressed from the endogenous gene encoding said polypeptide or protein.

A "2-oxogluatarate decarboxylase" protein catalyzes the conversion of 2-oxoglutarate to succinate semialdehyde.

A "phosphotransacylase" protein catalyzes the transfer of a phosphorylated acyl group to CoA according to the reaction:

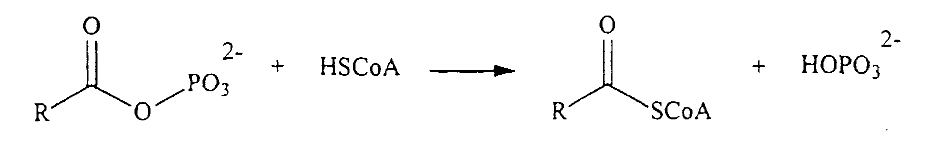

The phrases "polyhydroxyalkanoic acid biosynthetic genes" and "polyhydroxyalkanoic acid biosynthetic enzymes" refer to those genes or enzymes leading to anabolic reactions in the pathway of polyhydroxyalkanoic acid production.

The phrase "polyhydroxyalkanoate (PHA) synthase" refers to enzymes that convert hydroxyacyl-CoAs to polyhydroxyalkanoates and free CoA.

The term "promoter" or "promoter functional in bacteria" refer to a nucleotide sequence, usually found upstream (5') to a coding sequence, that controls expression of the coding sequence by controlling production of messenger RNA (mRNA) by providing the recognition site for RNA polymerase and/or other factors necessary for the start of transcription at the correct site. The promoters disclosed herein, and biologically functional equivalents thereof, are responsible for driving the transcription of coding sequences under their control when introduced into a bacterial cell, as demonstrated by their ability to produce mRNA.

The terms "recombinant vector" and "vector" refer to any agent such as a plasmid, cosmid, virus, autonomously replicating sequence, phage, or linear or circular single-stranded or double-stranded DNA or RNA nucleotide sequence, derived from any source, capable of genomic integration or autonomous replication, comprising a DNA molecule in which one or more DNA sequences have been linked in a functionally operative manner ("operatively linked"). Such recombinant DNA constructs or vectors are capable of introducing a 5' regulatory sequence or promoter region and a DNA sequence for a selected gene product into a cell in such a manner that the DNA sequence is transcribed into a functional mRNA which is translated and therefore expressed.

A "succinate-semialdehyde dehydrogenase" protein catalyzes the conversion of succinyl-CoA to succinate semialdehyde.

A "succinate:acetyl-CoA transferase" protein catalyzes the conversion of succinate to succinyl-CoA.

### DETAILED DESCRIPTION OF ILLUSTRATIVE EMBODIMENTS

The present invention provides a novel method for the preparation of polyester materials. In one important embodiment, co-expression of a recombinant polyhydroxyalkanoic acid synthase gene and a recombinant fatty acid:acyl-CoA transferase gene in a cell enable the biosynthesis of poly (4-hydroxybutyric acid). In an alternative embodiment, the co-expression of a recombinant polyhydroxyalkanoic acid synthase gene and a recombinant fatty acid:acyl-CoA transferase gene in bacteria leads to the biosynthesis of poly (3-hydroxybutyric acid). In a further alternative embodiment, co-expression of a recombinant polyhydroxyalkanoic acid synthase gene and a recombinant fatty acid:acyl-CoA transferase gene in a cell enable the biosynthesis of the copolyester poly (3-hydroxybutyric acid -co- 4-hydroxybutyric acid). In an alternative embodiment, co-expression of a recombinant polyhydroxyalkanoic acid synthase gene and a recombinant fatty acid:acyl-CoA transferase gene in a cell enable the biosynthesis of poly (4-hydroxybutyric acid).

The present application also describes, the co-expression of a polyhydroxyalkanoic acid synthase gene and an acyl-CoA synthetase gene in a cell, which leads to the biosynthesis of poly (3-hydroxybutyric acid). Thus, co-expression of a polyhydroxyalkanoic acid synthase gene and an acyl-CoA synthetase gene in a cell enable the biosynthesis of the copolyester poly (3-hydroxybutyric acid -co- 4-hydroxybutyric acid). Co-expression of a polyhydroxyalkanoic acid synthase gene and an acyl-CoA synthetase gene in a cell enable the biosynthesis of poly (4-hydroxybutyric acid).

In one important embodiment, the invention provides a nucleic acid segment that encodes a recombinant polyhydroxyalkanoic acid synthase protein, and that encodes a recombinant fatty acid:acyl-CoA transferase protein. The polyhydroxyalkanoic acid synthase protein may generally be any polyhydroxyalkanoic acid synthase protein suitable for the production of polyester materials according to the inventive processes. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Alcaligenes sp.*, *Rhizobium etli*, *Paracoccus denitrificans*, *Acinetobacter sp.*, *Rhodobacter sphaeroides*, *Methylobacterium extorquens*, *Pseudomonas oleovorans*, *Pseudomonas aeruginosa*, *Rhodococcus ruber*, and *Zoogloea ramigera*. Dimeric polyhydroxyalkanoic acid synthase proteins include, but are not limited to, those from *Thiocystis violacea* and *Chromatium vinosum.* Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The fatty acid:acyl-CoA transferase protein may be any fatty acid:acyl-CoA transferase protein suitable for the preparation of polyester materials according to the inventive processes. Fatty acid:acyl-CoA transferase proteins include, but are not limited to, 4-hydroxybutyrate:acetyl-CoA transferase from *Clostridium aminobutyricum*, propionate:acyl-CoA transferase from *Clostridium propionicum*, and succinate:acyl-CoA transferase from *Clostridium kluyveri*. Preferably, the fatty acid:acyl-CoA transferase protein is a 4-hydroxybutyrate:acyl-CoA transferase protein, more preferably a *Clostridium kluyveri* 4-hydroxybutyrate:acyl-CoA transferase protein, and most preferably, the *Clostridium kluyveri* 4-hydroxybutyrate:acyl-CoA transferase protein encoded by the *Clostridium kluyveri orfZ* 4-hydroxybutyrate:acyl-CoA transferase structural gene. The nucleic acid segment may comprise deoxyribonucleic acids (i.e. DNA) or ribonucleic acids (i.e. RNA). The nucleic acid segment may further be single or double stranded. The nucleic acid segment may further be linear or circular in conformation. The nucleic acid segment may further comprise a promoter sequence functional in bacterial cells. The promoter may be inducible or constitutive. Promoters functional in bacterial cells are generally known to those of skill in the art, and include, but are not limited to the lac promoter, the bla promoter, the P_{L} promoter, the P_{trc} promoter, and the T7 promoter.

The present application also describes a nucleic acid segment that encodes a polyhydroxyalkanoic acid synthase protein, and that encodes an acyl-CoA synthetase protein. The polyhydroxyalkanoic acid synthase protein may generally be any polyhydroxyalkanoic acid synthase protein suitable for the production of polyester materials according to the inventive processes. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Alcaligenes sp.*, *Rhizobium etli*, *Paracoccus denitrificans*, *Acinetobacter sp.*, *Rhodobacter sphaeroides*, *Methylabacterium extorquens*, *Pseudomonas oleovorans*, *Pseudomonas aeruginosa*, *Rhodococcus ruber*, and *Zoogloea ramigera*. Dimeric polyhydroxyalkanoic acid synthase proteins include, but are not limited to, those from *Thiocystis violacea* and *Chromatium vinosum.* Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The acyl-CoA synthetase protein may be any acyl-CoA synthetase protein suitable for the preparation of polyester materials according to the inventive processes. Sources of acyl-CoA synthetase proteins include, but are not limited to, *Alcaligenes eutrophus, Methanothrix soehngenii*, and *Aspergillus nidulans*. Preferably, the acyl-CoA synthetase protein is a thiokinase protein, and more preferably, a 4-hydroxybutyrate thiokinase protein. The nucleic acid segment may comprise deoxyribonucleic acids (i.e. DNA) or ribonucleic acids (i.e. RNA). The nucleic acid segment may further be single or double stranded. The nucleic acid segment may further be linear or circular in conformation. The nucleic acid segment may further comprise a promoter sequence functional in bacterial cells. The promoter may be inducible or constitutive. Promoters functional in bacterial cells are generally known to those of skill in the art, and include, but are not limited to the lac promoter, the bla promoter, the P_{L} promoter, the P_{trc} promoter, and the T7 promoter.

The invention further provides recombinant vectors comprising a nucleic acid segment, wherein the segment encodes a polyhydroxyalkanoic acid synthase protein and encodes a fatty acid:acyl-CoA transferase protein. This vector may generally be any vector suitable for the delivery of the nucleic acid into a cell. Vectors are well known to those of skill in the art, and include, but are not limited to, plasmids, artificial chromosomes, viruses, bacteriophage, cosmids, and phagemids. In a preferred embodiment, the vector may be pKSSE5.3 or pSKSE5.3 as disclosed herein.

In a preferred embodiment, the invention encompasses a cell comprising a nucleic acid segment encoding a recombinant polyhydroxyalkanoic acid synthase protein and encoding a recombinant fatty acid:acyl-CoA transferase protein. The polyhydroxyalkanoic acid synthase protein may generally be any polyhydroxyalkanoic acid synthase protein suitable for the production of polyester materials according to the inventive processes. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Alcaligenes sp.*, *Rhizobium etli*, *Paracoccus denitrificans*, *Acinetobacter sp.*, *Rhodobacter sphaeroides*, *Methylobacterium extorquens*, *Pseudomonas oleovorans*, *Pseudomonas aeruginosa*, *Rhodococcus ruber*, and *Zoogloea ramigera*. Dimeric polyhydroxyalhanoic acid synthase proteins include, but are not limited to, those from *Thiocystis violacea* and *Chromatium vinosum.* Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The fatty acid:acyl-CoA transferase protein may be any fatty acid:acyl-CoA transferase protein suitable for the preparation of polyester materials according to the inventive processes. Fatty acid:acyl-CoA transferase proteins include, but are not limited to, 4-hydroxybutyrate:acyl-CoA transferase from *Clostridium aminobutyricum*, propionate:acyl-CoA transferase from *Clostridium propionicum*, and succinate:acyl-CoA transferase from *Clostridium kluyveri*. Preferably, the fatty acid:acyl-CoA transferase protein is a 4-hydroxybutyrate:acyl-CoA transferase protein, more preferably a *Clostridium kluyveri 4*-hydroxybutyrate:acyl-CoA transferase protein, and most preferably, the *Clostridium kluyveri* 4-hydroxybutyrate:acyl-CoA transferase protein encoded by the *Clostridium kluyveri orfZ 4*-hydroxybutyrate:acyl-CoA transferase structural gene. The cell may generally be any cell suitable for the preparation of polyester materials. The cell may be, but is not limited to, a plant cell, a mammalian cells, an insect cell, a fungal cell, and a bacterial cell. Preferably, the cell is a plant cell. Preferably, the bacterial cell is *Escherichia coli*, and more preferably, the bacterial cell is *Escherichia coli* strain XL1-Blue.

We also describe a cell comprising a nucleic acid segment encoding a polyhydroxyalkanoic acid synthase protein and encoding an acyl-CoA synthetase protein. The polyhydroxyalkanoic acid synthase protein may generally be any polyhydroxyalkanoic acid synthase protein suitable for the production of polyester materials according to the inventive processes. The polyhydroxyalkanoic acid synthase protein may be monomeric or dimeric. Monomeric polyhydroxyalkanoic acid synthase proteins include, but are not limited to, those from *Rhizobium meliloti*, *Alcoligenes eutrophus*, *Alcaligenes sp.*, *Rhizobium etli*, *Paracoccus denitrificans*, *Acinetobacter sp.*, *Rhodobacter sphaeroides*, *Methylobacrerium extorquens*, *Pseudomonas oleovorans*, *Pseudomonas aeruginosa*, *Rhodococcus ruber*, and *Zoogloea ramigera*. Dimeric polyhydroxyalkanoic acid synthase proteins include, but are not limited to, those from *Thiocystis violacea* and *Chromatium vinosum*. Preferably, the polyhdroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The acyl-CoA synthetase protein may be any acyl-CoA synthetase protein suitable for the preparation of polyester materials according to the inventive processes. Sources of acyl-CoA synthetase proteins include, but are not limited to, *Alcaligenes eutrophus*, *Methanothrix soehngenii*, and *Aspergillus nidulans*. Preferably, the acyl-CoA synthetase protein is a thiokinase protein, and more preferably a 4-hydroxybutyrate thiokinase protein. The cell may generally be any cell suitable for the preparation of polyester materials. The cell may be, but is not limited to, a plant cell, a mammalian cells, an insect cell, a fungal cell, and a bacterial cell. Preferably, the cell is a plant cell. Preferably, the bacterial cell is *Escherichia coli*, and more preferably, the bacterial cell is *Escherichia coli* strain XL1-Blue.

The invention further discloses methods for the preparation of a transformed cell, the transformed cell being suitable for the preparation of polyester materials. The type of cell includes, but is not limited to, a plant cell, a mammalian cell, an insect cell, a fungal cell, and a bacterial cell. Preferably, the cell is a plant cell. Alternatively, the cell is preferably a bacterial cell, more preferably the bacterial cell is *Escherichia coli*, and most preferably, the bacterial cell is *Escherichia coli* strain XL1-Blue. Means for the transformation of plants are well known in the art. Methods include, but are not limited to, liposome mediated transformation, electroporation, treatment with chemicals that increase free DNA uptake, free DNA delivery via microparticle bombardment,and transformation using viruses or pollen. Means for the transformation of bacterial cells are also well known in the art. Methods include, but are not limited to, electroporation, calcium chloride mediated transformation, and polyethylene glycol mediated transformation. The disclosed transformation methods comprise selecting a host cell, contacting the host cell with a nucleic acid segment encoding a polyhydroxyalkanoic acid synthase protein and encoding a fatty acid:acyl-CoA transferase protein, the contacting step being performed under conditions suitable for uptake of the nucleic acid segment by the cell. Subsequent regeneration of the cell affords the transformed cell. The polyhydroxyalkanoic acid synthase protein may generally be any polyhydroxyalkanoic acid synthase protein suitable for the production of polyester materials according to the inventive processes. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Alcaligenes sp.*, *Rhizobium etli*, *Paracoccus denitrificans*, *Acinetobacter sp.*, *Rhodobacter sphaeroides*, *Methylobacterium extorquens*, *Pseudomonas oleovorans*, *Pseudomonas aeruginosa*, *Rhoducoccus ruber*, and *Zoogloea ramigera*. Dimeric polyhydroxyalkanoic acid synthase proteins include, but are not limited to, those from *Thiocystis violacea* and *Chromatium vinosum*. Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The fatty acid:acyl-CoA transferase protein may be any fatty acid:acyl-CoA transferase protein suitable for the preparation of polyester materials according to the inventive processes. Fatty acid:acyl-CoA transferase proteins include, but are not limited to, 4-hydroxybutyrate:acetyl-CoA acyltransferase from *Clostridium aminobutyricum*, propionate:acetyl-CoA acyltransferase from *Clostridium propionicum*, and succinate:acetyl-CoA transferase from *Clostridium kluyveri*. Preferably, the fatty acid:acyl-CoA transferase protein is a 4-hydroxybutyrate:acyl-CoA transferase protein, more preferably a *Clostridium kluyveri* 4-hydroxybutyrate:acyl-CoA transferase protein, and most preferably, the *Clostridium kluyveri* 4-hydroxybutyrate:acyl-CoA transferase protein encoded by the *Clostridium kluyveri orfZ* 4-hydroxybutyrate:acyl-CoA transferase structural gene.

The invention discloses alternative methods for the preparation of a transformed cell, the transformed cell being suitable for the preparation of polyester materials. The disclosed transformation methods comprise selecting a host cell, contacting the host cell with a nucleic acid segment encoding a polyhydroxyalkanoic acid synthase protein and encoding an acyl-CoA synthetase protein, the contacting step being performed under conditions suitable for uptake of the nucleic acid segment by the cell. Subsequent regeneration of the cell affords the transformed cell. The type of cell includes, but is not limited to, a plant cell, a mammalian cell, an insect cell, a fungal cell, and a bacterial cell. Preferably, the cell is a plant cell. Alternatively, the cell is preferably a bacterial cell, more preferably the bacterial cell is *Escherichia coli*, and most preferably, the bacterial cell is *Escherichia coli* strain XL1-Blue. Means for the transformation of plants are well known in the art. Methods include, but are not limited to, liposome mediated transformation, electroporation, treatment with chemicals that increase free DNA uptake, free DNA delivery via microparticle bombardment, and transformation using viruses or pollen. Means for the transformation of bacterial cells are also well known in the art. Methods include, but are not limited to, electroporation, calcium chloride mediated transformation, and polyethylene glycol mediated transformation. The polyhydroxyalkanoic acid synthase protein may generally be any polyhydroxyalkanoic acid synthase protein suitable for the production of polyester materials according to the inventive processes. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Acaligenes sp.*, *Rhizobium etli*, *Paracoccus denitrificans*, *Acinetobacter sp.*, *Rhodobacter sphaeroides*, *Methylobacterium extorquens*, *Pseudomonas oleovorans*, *Pseudomonas aeruginosa*, *Rhodococcus ruber*, and *Zoogloea ramigera*. Dimeric polyhydroxyalkanoic acid synthase proteins include, but are not limited to, those from *Thiocystis violacea* and *Chromatium vinosum*. Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The acyl-CoA synthetase protein may be any acyl-CoA synthetase protein suitable for the preparation of polyester materials according to the inventive processes. Sources of acyl-CoA synthetase proteins include, but are not limited to, *Alcaligenes eutrophus*, *Methanothrix soehngenii*, and *Aspergillus nidulans*. Preferably. the acyl-CoA synthetase protein is a thiokinase protein, and more preferably, a 4-hydroxybutyrate thiokinase protein.

The invention discloses methods for the preparation of polyester materials. The polyester materials may be any polyester material obtained via the inventive processes. The polyester may be a homopolyester or a copolyester. Preferably, the homopolyester is poly (4-hydroxybutyric acid) or poly (3-hydroxybutyric acid). The copolyester is preferably poly (3-hydroxybutyric acid -co- 4-hydroxybutyric acid). In a preferred embodiment, the method comprises the steps of a) obtaining a cell containing a nucleic acid segment, the nucleic acid segment encoding a polyhydroxyalkanoic acid synthase protein and encoding a fatty acid:acyl-CoA transferase protein; b) establishing a culture of the cell; c) culturing the cell under conditions suitable for the production of a polyester: and d) isolating the polyester from the cell. The polyhydroxyalkanoic acid synthase protein may generally be any polyhydroxyalkanoic acid synthase protein suitable for the production of polyester materials according to the inventive method. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Alcaligenes sp.*, *Rhizobium etli*, *Paracoccus denitrificans*, *Acinetobacter sp.*, *Rhodobacter sphaeroides*, *Methylobacterium extorquens*, *Pseudomonas oleovorans*, *Pseudomonas aeruginosa*, *Rhodococcus ruber*, and *Zoogloea ramigera*. Dimeric polyhydroxyalkanoic acid synthase proteins include, but are not limited to, those from *Thiocystis violacea* and *Chromatium* *vinosum*. Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The fatty acid:acyl-CoA transferase protein may be any fatty acid:acyl-CoA transferase protein suitable for the preparation of polyester materials according to the inventive method. Sources of acyl-CoA synthetase proteins include, but are not limited to, *Alcaligenes eutrophus*, *Methanoihrix soehngenii*, and *Aspergillus nidulans*. Preferably, the fatty acid:acyl-CoA transferase protein is a 4-hydroxybutyrate:acyl-CoA transferase protein, more preferably a *Clostridium kluyveri* 4-hydroxybutyrate:acyl-CoA transferase protein, and most preferably, the *Clostridium kluyveri* 4-hydroxybutyrate:acyl-CoA transferase protein encoded by the *Clostridium kluyveri orfZ* 4-hydroxybutyrate:acyl-CoA transferase structural gene. The culture may contain glucose or any material capable of conversion to glucose by the cell, as a carbon source. The culture may contain 4-hydroxybutyric acid, the sodium salt of 4-hydroxybutyric acid, γ-butyrolactone, 1,4-butanediol, 4-hydroxyvaleric acid, γ-valerolactone, 1,4-pentanediol, 3-hydroxybutyric acid, the sodium salt of 3-hydroxybutyric acid, a hydroxypropionic acid, a hydroxybutyric acid, a hydroxyvaleric acid, a hydroxycaproic acid, a hydroxyheptanoic acid, a hydroxyoctanoic acid, a hydroxydecanoic acid, γ-caprolactone, γ-heptanoloactone, γ-octanolactone, γ-decanolactone, or any material capable of conversion to 4-hydroxybutyric acid by the cell. The culture may contain molecular oxygen. Molecular oxygen may be present due to bubbling of oxygen gas, or an oxygen containing gas, such as air, through the culture. Alternatively, the molecular oxygen may be present due to agitation of the culture. The cell may contain an protein capable of hydrolyzing lactones to the corresponding hydroxyalkanoic acids. Such proteins may include, but are not limited to, the 1,4-lactonase proteins from rat and humans. To facilitate conversion of 2-oxoglutarate to polyester materials, the cell may further comprise a 2-oxoglutarate decarboxylase protein (for example, from *Leuconosioc oenos* or *Euglena gracilis*) and a 4-hydroxybutyrate dehydrogenase protein (for example, from *C. Kluyveri* or *A. eutrophus*). To facilitate conversion of succinate to polyester materials, the cell may further comprise a succinate:acetyl-CoA transferase protein (for example, from *C. kluyveri*), a succinate-semialdehyde dehydrogenase protein (for example, from *C. kluyveri*), and a 4-hydroxybutyrate dehydrogenase protein. To facilitate conversion of succinyl-CoA to polyester materials, the cell may further comprise a succinate-semialdehyde dehydrogenase protein, and a 4-hydroxybutyrate dehydrogenase protein. To facilitate conversion of propionyl-CoA to polyester materials, the cell may further comprise a 2-methylcitrate synthase protein (for example, from *Saccharomyces cerevisiae*), a 2-methylcitrate dehyratase protein (for example, from *Saccharomyces cerevisiae*), a 2-methylisocitrate dehydratase protein (for example, from *Saccharomyces cerevisiae*), a 2-methylisocitrate lyase protein (for example, from *Saccharomyces cerevisiae*), a succinate:acetyl-CoA transferase protein (for example, from *C. kluyveri*), a succinate-semialdehyde dehydrogenase protein, and a 4-hydroxybutyrate dehydrogenase protein. It will be apparent to those of skill in the art that different combinations of polyhydroxyalkanoic acid synthases, fatty acid:acyl-CoA transferases, and chemical substrates can be used according to the inventive methods to afford polyester materials.

The invention further describes alternative methods for the preparation of polyester materials. In a preferred embodiment, the method comprises the steps of a) obtaining a cell containing a nucleic acid segment, the nucleic acid segment encoding a polyhydroxyalkanoic acid synthase protein and encoding an acyl-CoA synthetase protein; b) establishing a culture of the cell; c) culturing the cell under conditions suitable for the production of a polyester; and d) isolating the polyester from the cell. The polyester materials may be any polyester material obtained via the inventive processes. The polyester may be a homopolyester or a copolyester. Preferably, the homopolyester is poly (4-hydroxybutyric acid) or poly (3-hydroxybutyric acid). The copolyester is preferably poly (3-hydroxybutyric acid -co- 4-hydroxybutyric acid). The polyhydroxyalkanoic acid synthase protein may generally be any polyhydroxyalkanoic acid synthase protein suitable for the production of polyester materials according to the inventive method. The polyhydroxyalkanoic acid synthase protein may be monomeric or dimeric. Monomeric polyhydroxyalkanoic acid synthase proteins include, but are not limited to, those from *Rhizobium meliloti*, *Alcaligenes eutrophus*, *Alcaligenes sp.*, *Rhizobium etli*, *Paracoccus denitrificans*, *Acinetobacter sp.*, *Rhodobacter sphaeroides*, *Methylobacterium extorquens*, *Pseudomonas oleovorans*, *Pseudomonas aeruginosa*, *Rhodococcus ruber*, and *Zoogloea ramigera*. Dimeric polyhydroxyalkanoic acid synthase proteins include, but are not limited to, those from *Thiocystis violacea* and *Chromatium vinosum*. Preferably, the polyhydroxyalkanoic acid synthase protein is an *Alcaligenes eutrophus* polyhydroxyalkanoic acid synthase protein, and more preferably, the *Alcaligenes eutrophus* polyhydroxyalkanoic acid synthase protein encoded by the *Alcaligenes eutrophus phaC* polyhydroxyalkanoic acid synthase structural gene. The acyl-CoA synthetase protein may be any acyl-CoA synthetase protein suitable for the preparation of polyester materials according to the inventive processes. Sources of acyl-CoA synthetase proteins include, but are not limited to, *Alcaligenes eutrophus*, *Methanothrix soehngenii*, and *Aspergillus nidulans*. Preferably, the acyl-CoA synthetase protein is a thiokinase protein, and more preferably, a 4-hydroxybutyrate thiokinase protein. The culture may contain glucose or any material capable of conversion to glucose by the cell, as a carbon source. The culture may contain 4-hydroxybutyric acid, the sodium salt of 4-hydroxybutyric acid, γ-butyrolactone, 1,4-butanediol. 4-hydroxyvaleric acid, γ-valerolactone, 1,4-pentanediol, 3-hydroxybutyric acid, the sodium salt of 3-hydroxybutyric acid, a hydroxypropionic acid, a hydroxybutyric acid, a hydroxyvaleric acid, a hydroxycaproic acid, a hydroxyheptanoic acid, a hydroxyoctanoic acid, a hydroxydecanoic acid, γ-caprolactone, γ-heptanoloactone, γ-octanolactone, γ-decanolactone, or any material capable of conversion to 4-hydroxybutyric acid by the cell. The culture may contain molecular oxygen. Molecular oxygen may be present due to bubbling of oxygen gas, or an oxygen containing gas, such as air, through the culture. Alternatively, the molecular oxygen may be present due to agitation of the culture. The cell may contain an enzyme capable of hydrolyzing lactones to the corresponding hydroxyalkanoic acids. Such proteins may include, but are not limited to, the 1,4-lactonase proteins from rat and humans. To facilitate conversion of 2-oxoglutarate to polyester materials, the cell may further comprise a 2-oxoglutarate decarboxylase protein and a 4-hydroxybutyrate dehydrogenase protein. To facilitate conversion of succinate to polyester materials, the cell may further comprise a succinate:acetyl-CoA transferase protein, a succinate-semialdehyde dehydrogenase protein, and a 4-hydroxybutyrate dehydrogenase protein. To facilitate conversion of succinyl-CoA to polyester materials, the cell may further comprise a succinate-semialdehyde dehydrogenase protein, and a 4-hydroxybutyrate dehydrogenase protein. To facilitate conversion of propionyl-CoA to polyester materials, the cell may further comprise a 2-methylcitrate synthase protein, a 2-methylcitrate dehyratase protein, a 2-methylisocitrate dehydratase protein, a 2-methylisocitrate lyase protein, a succinate:acetyl-CoA transferase protein, a succinate-semialdehyde dehydrogenase protein, and a 4-hydroxybutyrate dehydrogenase protein. It will be apparent to those of skill in the art that different combinations of polyhydroxyalkanoic acid synthases, acyl-CoA synthetases, and chemical substrates can be used according to the inventive methods to afford polyester materials.

In a further alternative embodiment, the preparation of polyester materials may be achieved by the co-expression of a polyhydroxyalkanoic acid synthase protein, an acyl kinase protein, and a phosphotransacylase protein. A source of acyl kinase includes, but is not limited to, the butyrate kinase from *Clostridium acetobutylicum*. A source of a phosphotransacylase protein includes, but is not limited to, the phosphotransbutyrylase protein from *Clostridium acetobutylicum*.

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLES

### Materials and methods

### Microbiological Deposit

A deposit according to the Budapest Treaty of *Escherichia coli* XL1-Blue containing the recombinant plasmid pKSSE5.3 (accession number DSM 11427) and of *Escherichia coli* XL1-Blue containing the recombinant plasmid pSKSE5.3 (accession number DSM 11435) was made on February 24, 1997 with the Deutsche Sammlung von Mikroorganismen und Zellkulturen GmbH (DSMZ), Mascheroder Weg 1b, D-38124 Braunschweig, Germany.

### Bacterial strains and plasmids

*Escherichia coli* strain XL1-Blue (Bullock, W.O. et al. (1987) *BioTechniques* 5:376-378) and the plasmids pBluescriptKS⁻ and pBluescriptSK⁻ (Stratagene, La Jolla, CA), pSK2665 (Schubert, P. et al. (1991) *J. Bacteriol*. 173:168-175) and pCK3pSK (Söhling, B. and Gottschalk, G. (1996) *J. Bacteriol.* 178:871-880) were used.

### Media and cultivation conditions

Cells of *Escherichia coli* were cultivated at 37°C in complex Luria-Bertani broth or in M9-minerals salts medium supplemented with 0.02% (w/vol) yeast extract (Sambrook. J. et al. (1989) Molecular cloning; a laboratory manual. 2nd edition. Cold Spring Harbor Laboratory. Cold Spring Harbor, N.Y.). The addition of antibiotics prepared according to Sambrook, J. et al. (1989), and of carbon sources, which were sterilized by filtration, are described in the text. Cultivations were performed either on solidified media, which were obtained by the addition of 1.5% (wt/vol) agar, or in liquid media in erlenmeyer flasks incubated on a rotary shaker.

### Isolation and analysis of polyesters

For quantitative determination of polyhydroxyalkanoic acid and for the analysis of the constituents of polyhydroxyalkanoic acid, 3 - 5 mg of lyophilized cell material or the isolated polyester were subjected to methanolysis in the presence of 15% (vol/vol) sulfuric acid. The resulting hydroxyacyl methylesters were analyzed by gas chromatography as described in detail by Brandl, H. et al. (1988, *Appl. Environ. Microbiol.* 54:1977-1982) and Timm. A. et al. (1990, *Appl. Microbiol. Biotechnol*. 33:296-301).

Polyhydroxyalkanoic acids were isolated from lyophilized cells by extraction with chloroform in a soxhlet apparatus. The polyester was precipitated from the chloroform solution by the addition of 10 volumes of ethanol, and the precipitate was subsequently separated from the solvents by filtration. Remaining solvents were removed by exposure of the polyester to a stream of air. For further purification, the polyester was dissolved in chloroform and the precipitation with ethanol was repeated.

### Nucleic acid techniques

Plasmid DNA and DNA restriction fragments were isolated and analyzed by standard methods (Sambrook, J. et al. (1989) Molecular cloning; a laboratory manual, 2nd edition, Cold Spring Harbor Laboratory, Cold Spring Harbor, N.Y.). Restriction enzymes, ligases, and other enzymes were used according to the manufacturer's instructions.

### Physical characterization of poly(4-hydroxybutyric acid) samples

Analysis of polymer composition was performed using gas chromatography methods as described by Braunegg. G. et al. (1978, *Eur. J. Appl. Microbiol*. 6:29-37). Analysis of molecular weight and polydispersity was performed by gel permeation chromatography (GPC, Ishihara. Y. et al. (1996) *J. Ferm. Bioeng*. 81:422-428). Analysis of melting points, rate of crystallization, d*H*_{m,} and Eₐ were performed by differential scanning calorimetry (DSC, Kemnitzer. J.E. et al. (1995) *J. Env. Polym. Degrad*. 3:37-47).

### EXAMPLE 1: Construction of plasmids

A 3.5-kbp *Sma*I/*Apa*I restriction fragment comprising the entire polyhydroxyalkanoic acid synthase structural gene (*phaC*_{*Ae*}, GenBank Accession number J05003, Peoples, O.P. and Sinskey, A.J. (1989) *J. Biol. Chem.* 264:15298-15303) plus 878 of 1.221 bp of the 5' region of the β-ketothiolase structural gene from *A. eutrophus*, referred to as SA35, was isolated from the hybrid plasmid pSK2665 that had been cloned previously (Schubert, P. et al. (1991) *J. Bacteriol*. 173:168-175). In addition, a 1.8 kb *Apa*I/*Eco*RI restriction fragment comprising the entire orfZ_{*Ck*} (*phaA'*_{*Ae*}, GenBank Accession number L21902, Söhling, B. and Gottschalk, G. (1993) *J. Bacteriol*. 178:871-880) from *C. kluyveri*, and referred to as AE18, was isolated from the hybrid plasmid pCK3pSK that had been cloned previously (Söhling, B. and Gottschalk, G. (1996) *J. Bacteriol*. 178:871-880). Both fragments were ligated to EcoRI/SmaI digested pBluescript vectors KS⁻ and SK⁻. The ligation products (pKSSE5.3 and pSKSE5.3, respectively) were transformed into *Escherichia coli* strain XL1-Blue using calcium chloride methodologies (Sambrook, J. et al. (1989) Molecular cloning; a laboratory manual. 2nd edition. Cold Spring Harbor Laboratory, Cold Spring Harbor, N.Y.). Plasmid pKSSE5.3 contains *phaC*_{*Ae*} and orfZ_{*Ck*} adjacent but anti linear to the *lacZ* promoter. In pSKSE5.3, *phaC*_{*Ae*} and orfZ_{*Ck*} were located downstream and colinear to the *lacZ* promoter. All constructs were analyzed by agarose gel electrophoresis for the presence of the expected restriction fragments.

For control experiments, fragment SA35 was ligated to pBluescript KS⁻ or to pBluescript SK⁻ resulting in the plasmids pKSSA35 (*phaC*_{*Ae*} plus *phaA'*_{Ae} adjacent but antilinear to the *lacZ* promoter) or pSKSA35 (*phaC*_{*Ae*} plus *phaA'*_{Ae} downstream and colinear to the *lacZ* promoter), respectively. Similarly, fragment AE18 alone was ligated to pBluescript KS⁻ or to pBluescript SK⁻ resulting in the plasmids pKSAE 18 (orfZ_{*Ck*} downstream and colinear to the *lacZ* promoter) and pSKAE 18 (orfZ_{*Ck*} adjacent but antilinear to the *lacZ* promoter), respectively.

### EXAMPLE 2: Synthesis of poly(4-hydroxybutyric acid) from 4-hydroxybutyric acid in recombinant strains of Escherichia coli

The recombinant strains of *Escherichia coli* XL 1-Blue were cultivated at 37°C as batch cultures in erlenmeyer flasks either using LB complex medium supplemented with 4-hydroxybutyric acid alone or in combination with glucose as an additional carbon source, or M9 mineral salts medium containing 4-hydroxybutyric acid and glucose as carbon sources. The hybrid plasmids pKSSE5.3 and pSKSE5.3. which comprised *phaC*_{*Ae*}*, phaA'*_{Ae} plus orfZ _{*Ck*}, conferred to *Escherichia coli* the capability to synthesize and accumulate a homopolyester of 4-hydroxybutyric acid if the strains were cultivated in the presence of 4-hydroxybutyric acid plus glucose as carbon source (Table 1).

**Table 1.**

| Accumulation of poly(4-hydroxybutyric acid) by recombinant strains of *Escherichia coli* XL1-Blue from 4-hydroxybutyric acid plus glucose | | | | |
|---|---|---|---|---|
| Medium | Carbon source | volume of medium (mL) | PHA content (% of CDW) | accumulated polyester |
| with pSKSE5.3 | | | | |
| LB + IPTG | 0.5% glucose | 50 | 3.3 | poly (4HB) |
| | 0.4% 4HB (sodium salt) | | | |
| LB + IPTG | 0.5% glucose | 100 | 10.5 | poly (4HB) |
| | 0.4% 4HB (sodium salt) | | | |
| LB + IPTG | 0.5% glucose | 150 | 8.8 | poly (4HB) |
| | 0.4% 4HB (sodium salt) | | | |
| M9 + IPTG | 0.5% glucose | 100 | 16.9 | poly (4HB) |
| | 0.4% 4HB (sodium salt) | | | |

| with pKSSE5.3 | | | | |
|---|---|---|---|---|
| LB | 0.5% glucose | 50 | 4.3 | poly (4HB) |
| | 0.4% 4HB (sodium salt) | | | |
| LB | 0.5% glucose | 100 | 40.8 | poly (4HB) |
| | 0.4% 4HB (sodium salt) | | | |
| LB | 0.5% glucose | 150 | 20.4 | poly (4HB) |
| | 0.4% 4HB (sodium salt) | | | |
| M9 | 0.5% glucose | 100 | 58.5 | poly (4HB) |
| | 0.4% 4HB (sodium salt) | | | |

| with pSKSA35 | | | | |
|---|---|---|---|---|
| LB + IPTG | 0.5% glucose | 50-150 | nd | - |
| | 0.4% 4HB (sodium salt) | | | |
| M9 + IPTG | 0.5% glucose | 100 | nd | - |
| | 0.4% 4HB (sodium salt) | | | |
| with pKSAE18 | | | | |
| LB + IPTG | 0.5% glucose | 50-150 | nd | - |
| | 0.4% 4HB (sodium salt) | | | |
| M9 + IPTG | 0.5% glucose | 100 | nd | - |
| | 0.4% 4HB (sodium salt) | | | |
| Cells were cultivated at 37°C for 72 hours in one-stage cultivation experiments in 250 mL erlenmeyer flasks as described in the text that were incubated on a rotary shaker (220 rpm). The cultures were inoculated with 0.04 vol of an overnight preculture in Luria Bertani broth containing 0.5% (wt/vol) glucose plus 0.4% (wt/vol) 4-hydroxybutyric acid. M9 medium was supplemented with 0.02% (wt/vol) yeast extract. At the end of the experiment, the cells were harvested, washed with tap water, lyophilized and analyzed for PHA content and composition by gas chromatography. | | | | |
| Abbreviations and symbols: IPTG, isopropyl-1-thio-β-D-galactopyranoside; nd, not detectable; -, not relevant. | | | | |

Light microscopic examination of the cells revealed cytoplasmic inclusions, and the harvested cells were more opaque in appearance than were strains not harboring pKSSE5.3 or pSKSE5.3. If glucose was omitted as a cosubstrate, the amount of polyhydroxyalkanoic acid in the cells was approximately in the same range but consisting of a copolyester of 3-hydroxybutyric acid and 4-hydroxybutyric acid, poly(3-hydroxybutyric acid-*co*-4-hydroxybutyric acid), rather than poly(4-hydroxybutyric acid). Early in the growth, only 4-hydroxybutyric acid was incorporated into the polyhydroxyalkanoic acid. However, after approximately one day of cultivation, the cells began to incorporate increasing amounts of 3-hydroxybutyric acid, and after four days, the molar fraction of 3-hydroxybutyric acid had increased to approximately 70% (Table 2). If glucose was provided as the sole carbon source, no polyhydroxyalkanoic acid was accumulated. The addition of 4-hydroxybutyric acid to the medium as a carbon source appeared essential to obtain poly(4-hydroxybutyric acid), and glucose provided as a cosubstrate appeared to limit the incorporation of 3-hydroxybutyric acid into the polyhydroxyalkanoic acid.

**Table 2.**

| Accumulation of poly(3-hydroxybutyric acid-*co*-4-hydroxybutyric acid) by recombinant strains of *Escherichia coli* XL1-Blue from 4-hydroxybutyric acid | | | | |
|---|---|---|---|---|
| Strain and Carbon source(s) | Incubation time (hours) | PHA content (% of CDW) | PHA composition mole % 3HB | PHA composition mole % 4HB |
| E. coli XL1-Blue (pSKSE5.3) | | | | |
| in LB medium with | 20 | 13.3 | nd | 100 |
| 0.5% glucose and | 44 | 13.1 | 11 | 89 |
| 0.4% Na-4-hydroxybutyrate | 72 | 11.1 | 55 | 45 |
| | 94 | 15.1 | 64 | 36 |
| | | | | |
| in LB medium with | 20 | 20.2 | nd | 100 |
| 0.4% Na-4-hydroxybutyrate | 44 | 20.6 | 72 | 28 |
| | 72 | 10.2 | 61 | 39 |
| | 94 | 21.9 | 66 | 34 |

| E. coil XL1-Blue (pKSSE5.3) | | | | |
|---|---|---|---|---|
| in LB medium with | 20 | 14.6 | nd | 100 |
| 0.5% glucose and | 44 | 13.6 | nd | 100 |
| 0.4% Na-4-hydroxybutyrate | 72 | 7.0 | nd | 100 |
| | 94 | 15.9 | 7 | 93 |
| | | | | |
| in LB medium with | 20 | 22.9 | nd | 100 |
| 0.4% Na-4-hydroxybutyrate | 44 | 21.4 | 62 | 38 |
| | 72 | 23.7 | 63 | 37 |
| | 94 | 29.8 | 64 | 36 |
| Cells were cultivated at 37°C in 50 mL Luria Bertani complex medium, which contained the indicated carbon source, in 300 mL Erlenmeyer flasks. At the indicated times, 10 mL samples were withdrawn and subjected to gas chromatographic analysis of the polyester content and composition. | | | | |
| Abbreviations and symbols: nd, not detectable; PHA, polyhydroxyalkanoic acids: 3HB, 3-hydroxybutyric acid; 4HB. 4-hydroxybutyric acid; LB Luria Bertani; CDW, cellular dry weight | | | | |

Gas chromatographic analysis of the derivatives obtained from washed and lyophilized whole cells gave two major compounds in the chromatograms exhibiting retention times of 20.1 and 9.3 and one minor compound exhibiting a retention time of 24.1 min. These compounds represented most probably γ-butyrolactone, the methylester of 4-hydroxybutyric acid and the methyl ether of 4-hydroxybutyric acid, respectively, that were also obtained if only 4-hydroxybutyric acid were subjected to acidic methanolysis.

The amount of poly(4-hydroxybutyric acid) accumulated by the cells depended upon the plasmid present in the recombinant *Escherichia coli* XL1-Blue, the medium, and the cultivation conditions. Recombinant strains harboring plasmid pKSSE5.3 accumulated significantly more poly(4-hydroxybutyric acid) as compared to those harboring pSKSE5.3 in LB complex medium as well as in M9 mineral salts medium (Table 1). The addition of IPTG to cultures of *Escherichia coli* XL1-Blue harboring pSKSE5.3 did not significantly influence the amount of polyester produced. With either plasmid, the amount of poly(4-hydroxybutyric acid) accumulated by the cells was always higher in M9-mineral salts medium than in LB complex medium. Supplementation of oxygen to the cultures seems to be crucial for the amount of poly(4-hydroxybutyric acid) accumulated by the cells. This became obvious from experiments in which the ratio of the medium volume to the volume of the erlenmeyer flask was varied. When experiments were performed in a 250 mL erlenmeyer flask, the amount of poly(4-hydroxybutyric acid) increased tremendously when the volume of the medium was increased from 50 to 100 mL independently whether the recombinant strains harbored pSKSE5.3 or pKSSE5.3 (Table 1). If the volume of the medium was further increased to 150 mL. the amount of accumulated poly(4-hydroxybutyric acid) decreased.

All plasmids, which contained only *phaC*_{*Ae*} plus *phaA'*_{Ae} (pKSSA35 and pSKSA35) or only orfZ_{*Ck*} (pKSAE18 and pSKAE18), did not confer to *Escherichia coli* the capability to synthesize detectable poly(4-hydroxybutyric acid) or of any other polyhydroxyalkanoic acid if 4-hydroxybutyric acid or glucose alone or in combination were used a carbon sources and independent from the volume of the medium or whether IPTG was added or not.

None of the recombinant strains of *Escherichia coli* obtained in this study were able to grow in liquid or on solidified M9 minerals salts medium containing 4-hydroxybutyric acid as the sole carbon source.

### EXAMPLE 3: Properties of poly(4-hydroxybutyric acid) isolated from recombinant Escherichia coli

In order to confirm accumulation of poly(4-hydroxybutyric acid) by the recombinant strains and to isolate the polyester from the cells, *Escherichia coli* XL1-Blue (pKSSE5.3) was cultivated in M9 mineral salts medium on a larger scale. 2 L baffled erlenmeyer flasks containing 1700 mL medium supplemented with 0.4% (wt/vol) sodium 4-hydroxybutyrate and 1% or 2% glucose were inoculated with a preculture of 50 mL cells and incubated on a rotary shaker for 72 hours. This afforded approximately 1.9 g or 2.5 g, respectively, of dried cells. Gas chromatographic analysis of the whole cells indicated a poly(4-hydroxybutyric acid) content of approximately 80% (wt/wt). From these cells, 0.8 g or 1.1 g of polyhydroxyalkanoic acid could be extracted with chloroform and precipitated with ethanol, respectively. Therefore, only 52% or 55% of the polyester were recovered from the cells. This discrepancy could be explained by incomplete extractions of the cells since the extracted cells still contained poly(4-hydroxybutyric acid) and by losses during the precipitation of the polymer. The isolated material gave in the gas chromatogram only those signals indicative of 4-hydroxybutyric acid. Thus it was confirmed that the cells had accumulated poly(4-hydroxybutyric acid) homopolyester.

Poly(4-hydroxybutyric acid) produced by a polyhydroxyalkanoic acid -leaky mutant of *A. eutrophus* JMP222 and recombinant strains of this mutant harboring extra copies of the *A. eutrophus* PHA synthase operon (Steinbüchel, A. et al. (1994) *J. Environ. Polymer Degrad*. 2:67-74) were observed to be extracted from lyophilized cells at a lower rate than was poly (3-hydroxybutyric acid). Similarly, the poly(4-hydroxybutyric acid) produced by the recombinant strain of *Escherichia coli* employed in this study was extracted from lyophilized cells at a much lower rate than, for example poly(3-hydroxybutyric acid). Poly(4-hydroxybutyric acid) from the recombinant strain precipitated in the presence of an excess of ethanol from the chloroform solution as a highly fibrous material which easily and almost quantitatively ended up on a glass rod if the latter was used for stirring during precipitation. Poly(4-hydroxybutyric acid) from the strains of *A. eutrophus* JMP222 (Steinbüchel, A. et al. (1994) *J. Environ. Polymer Degrad.* 2:67-74) was isolated as a white and elastic material.

Gel permeation chromatography experiments performed with two samples of poly(4-hydroxybutyric acid) homopolyester isolated from two independent batches of cells of recombinant strains of *E. coli* revealed molecular weights (*M*_{w}) of 1.75 x 10⁶ and 1.85 x 10⁶, respectively, with relative low polydispersities (*M*_{w}/*M*ₙ) of 1.45 and 1.48, respectively. Therefore, the molecular weights of these poly(4-hydroxybutyric acid) samples were significantly higher than the molecular weights of poly(4-hydroxybutyric acid) isolated from strains of *A. eutrophus* JMP222. In addition, the polydispersity of these samples was much lower (Steinbüchel, A. et al. (1994) *J. Environ. Polymer Degrad.* 2:67-74). The two poly(4-hydroxybutyric acid) samples exhibited melting points (Tₘ) of 67.6 and 63.1°C. The values for d*H*ₘ and Eₐ for the two samples were 45.7 and 44.3 J/g or 69.8 and 83.8 KJ/mol, respectively. At 70°C both polyester samples exhibited a slow rate for crystallization (> 30 min).

### EXAMPLE 4: Synthesis of polyhydroxyalkanoic acid from γ-butyrolactone, levulinic acid, 4-hydroxyvaleric acid or γ-valerolactone in recombinant strains of Escherichia coli

Carbon sources related to 4-hydroxybutyric acid were also investigated with respect to the formation of polyhydroxyalkanoic acid by the recombinant strains. For this purpose, cells were cultivated in two different stages. In the first stage the cells were grown for 64 hours in the complete M9 minerals salts medium containing 0.5% (wt/vol) glucose plus 0.1% (wt/vol) sodium 4-hydroxybutyrate as carbon sources. These cells were then washed with fresh M9 mineral salts medium and transferred to 250 mL erlenmeyer flasks containing 100 mL ammonium-free M9 mineral salts medium. The density of the cell suspension was diluted approximately 1:1 as compared to the density in the preculture. In the second stage the cells were cultivated for 72 hours on a rotary shaker. If *Escherichia coli* XL1-Blue (pKSSE5.3) was cultivated in M9 mineral salts medium containing 0.5% (wt/vol) glucose plus 0.4% (wt/vol) γ-butyrolactone as carbon sources, only low amounts of polyhydroxyalkanoic acid (usually below 10% of CDW, wt/wt) were accumulated (Table 3). However, these cells did not accumulate poly(4-hydroxybutyric acid) homopolyester but synthesized a copolyester consisting of 3-hydroxybutyric acid and 4-hydroxybutyric acid, with 4-hydroxybutyric acid as a minor constituent (usually below 30%, mol/mol).

**Table 3.**

| Cultivation of *E. coli* XL1-Blue (pKSSE5.3) on other precursor substrates for the incorporation of 4-hydroxyalkanoic acids in PHA | | | | | | |
|---|---|---|---|---|---|---|
| Medium | Carbon source | PHA content (% of CDW) | PHA composition (mole %) | | | |
| | | | 3HB | 3HV | 4HB | 4HV |
| two-stage cultivation experiments | | | | | | |
| M9* | 0.5% glucose | 9.6 | 71 | nd | 29 | nd |
| | 0.4% γ-butyrolactone | | | | | |
| M9* | 0.5% glucose | 4.2 | 100 | nd | nd | nd |
| | 0.4% Na-levulinate | | | | | |
| M9* | 0.5% glucose | 1.5 | 100 | nd | nd | nd |
| | 0.4% Na-4-hydroxyvalerate | | | | | |
| M9* | 0.5% glucose | 2.7 | 100 | nd | nd | nd |
| | 0.4% γ-valerolactone | | | | | |

| one-stage cultivation experiments | | | | | | |
|---|---|---|---|---|---|---|
| M9 | 0.5% glucose | 8.0 | nd | nd | 100 | nd |
| | 0.1% γ-butyrolactone | | | | | |
| M9 | 0.5% glucose | 13.9 | nd | nd | 100 | nd |
| | 0.2% γ-butyrolactone | | | | | |
| M9 | 0.5% glucose | 16.1 | nd | nd | 100 | nd |
| | 0.4% γ-butyrolactone | | | | | |
| M9 | 0.5% glucose | nd | nd | nd | nd | nd |
| | 0.2% Na-levulinate | | | | | |
| M9 | 0.5% glucose | nd | nd | nd | nd | nd |
| | 0.4% Na-levulinate | | | | | |

Cells were cultivated at 37°C for 72 hours in one- or two-stage cultivation experiments as described in the text. At the end of the experiment the cells were harvested, washed with tap water, lyophilized and analyzed for PHA content and composition by gas chromatography.

Abbreviations and symbols: M9, regular mineral salts medium supplemented with 0.02% (wt/vol) yeast extract; M9*, ammonium-free M9 mineral salts medium; nd, not detectable

If in these experiments γ-butyrolactone was replaced by 0.4% (wt/vol) sodium levulinate or by 0.2% (wt/vol) sodium 4-hydroxyvalerate or by 0.2% (wt/vol) γ-valerolactone, only very low amounts of polyhydroxyalkanoic acid never exceeding 4% of the CDW were synthesized and accumulated. Gas chromatographic analysis revealed 3-hydroxybutyric acid as the only constituent; 4-hydroxybutyric acid, 3-hydroxyvaleric acid (3HV) or 4-hydroxyvaleric acid (4HV) were not detected (Table 3).

Cells of *Escherichia coli* XL 1-Blue (pKSSE5.3) were also cultivated in single stage experiments as batch cultures in 250 mL erlenmeyer flasks containing 100 mL complete M9 minerals salts medium plus 0.5% /wt/vol) glucose plus 0.1 to 0.4% (wt/vol) γ-butyrolactone as carbon sources. The cells accumulated significantly more polyhydroxyalkanoic acid, and the polyester consisted of 4-hydroxybutyric acid as the only detectable constituent (Table 3). With 0.4% (wt/vol) sodium 4-hydroxybutyrate in the medium, for example, poly(4-hydroxybutyric acid) contributed 16.1% (wt/wt) to the CDW. If sodium levulinate instead of sodium 4-hydroxybutyrate was used as a second carbon source in addition to 0.5% (wt/vol) glucose, no polyhydroxyalkanoic acid was detected in the cells.

### EXAMPLE 5: Production of polyester materials in plants

In plants, transformation vectors capable of introducing bacterial genes involved in polyester biosynthesis can be designed. Generally, such vectors comprise one or more coding sequences of interest under the transcriptional control of 5' and 3' regulatory sequences, including a promoter, and a selectable marker. Typical regulatory sequences include a transcription initiation start site, an RNA processing signal, a transcription termination site, and/or a polyadenylation signal. Plant promoter can be inducible or constitutive, environmentally- or developmentally-regulated, or cell- or tissue-specific. Often-used promoters include the CaMV 35S promoter (Odell, J.T. et al. (1985) *Nature* 313:810-812), the enhanced CaMV 35S promoter, the Figwort Mosaic Virus (FMV) promoter (Richins, R.D. et al. (1987) *NAR* 20:8451-8466), the mannopine synthase (mas) promoter, the nopaline synthase (*nos*) promoter, and the octopine synthase (*ocs*) promoter. Useful inducible promoters include heat-shock promoters (Ou-Lee et al. (1986) *Proc. Natl. Acad. Sci. USA* 83:6815; a nitrate-inducible promoter derived from the spinach nitrite reductase gene (Back, E. et al. (1991) *Plant Mol. Biol*. 17:9-18); hormone-inducible promoters (Yamaguchi-, K. et al. (1990) *Plant Mol. Biol.* 15:905-912; Kares, C. et al. (1990) *Plant Mol. Biol.* 15:225-236), and light-inducible promoters associated with the small subunit of RuBP carboxylase and LHCP gene families (Kuhlemeier, C. et al. (1989) *Plant Cell* 1:471-478; Feinbaum, R.L. et al. (1991) *Mol. Gen. Genet.* 226:449-456; Weisshaar, B. et al. (1991) *EMBO J*. 10:1777-1786; Lam, E. and Chua, N.-H. (1990) *Science* 248:471-474; Castresana, C. et al. (1988) *EMBO J.* 7:1929-1936: Schulze-Lefert, P. et al. (1989) *EMBO J*. 8:651-656). Examples of useful tissue-specific, developmentally-regulated promoters include the β-conglycinin 7S promoter (Doyle, J.J. et al. (1986) *J. Biol. Chem*. 261:9228-9238; Slighton and Beachy (1987) *Planta* 172:356), and seed-specific promoters (Knutzon, D.S. et al. (1992) *Proc. Natl. Acad. Sci. USA* 89:2624-2628; Bustos, M.M. et al. (1991) *EMBO J*. 10: 1469-1480; Lam, E. and Chua, N.-H. (1991) *Science* 248:471-474; Stayton. M. et al. (1991) *Aust. J. Plant. Physiol.* 18:507-518). Plant functional promoters useful for preferential expression in seed plastids include those from plant storage protein genes and from genes involved in fatty acid biosynthesis in oilseeds. Examples of such promoters include the 5' regulatory regions from such genes as napin (Kridl et al. (1991) *Seed Sci. Res.* 1:209), phaseolin, zein, soybean trypsin inhibitor, ACP. stearoyl-ACP desaturase, and oleosin. Seed-specific gene regulation is discussed in EP 0 255 378. Promoter hybrids can also be constructed to enhance transcriptional activity (Hoffman, U.S. Patent No. 5,106,739), or to combine desired transcriptional activity and tissue specificity. Representative vectors often comprise, operatively linked in sequence in the 5' to 3' direction, a promoter sequence that directs the transcription of a downstream heterologous structural nucleic acid in a plant; optionally, a non-translated leader sequence; a nucleotide sequence that encodes a protein of interest: and a 3' non-translated region that encodes a polyadenylation signal which functions in plant cells to cause the termination of transcription and the addition of polyadenylate nucleotides to the 3' end of the mRNA encoding said protein. Additionally, a factor to consider is the timing and intracellular localization of proteins necessary for the biosynthesis of polyesters. For example, if fatty acid biosynthetic pathways are utilized in oilseed plants such as canola, then polyester biosynthetic protein expression should be concurrent with fatty acid biosynthesis and targeted to the seed leucoplast or leaf chloroplast.

A variety of different methods can be employed to introduce such vectors into plant protoplasts, cells, callus tissue, leaf discs, meristems, etcetera, to generate transgenic plants, including *Agrobacterium*-mediated transformation, particle gun delivery, microinjection, electroporation, polyethylene glycol-mediated protoplast transformation, liposome-mediated transformation, etcetera (reviewed in Potrykus, I. (1991) *Annu. Rev. Plant Physiol. Plant Mol. Biol*. 42:205-226). In general, transgenic plants comprising cells containing and expressing polyhydroxyalkanoic acid synthase and fatty acid:acyl-CoA transferase-encoding nucleic acid can be produced by transforming plant cells with a nucleic acid construct as described above via any of the foregoing methods; selecting plant cells that have been transformed on a selective medium; regenerating plant cells that have been transformed to produce differentiated plants; and selecting a transformed plant which expresses the polyhydroxyalkanoic acid synthase and fatty acid:acyl-CoA transferase -encoding nucleotide sequences.

The encoding nucleic acids can be introduced either in a single transformation event (all necessary DNAs present on the same vector), a co-transformation event (all necessary nucleic acids present on separate vectors that are introduced into plants or plant cells simultaneously), by independent transformation events (all necessary nucleic acids present on separate vectors that are introduced into plants or plant cells independently) or by re-transformation (transforming an already transformed line generated by a single transformation, co-transformation, or independent transformation events). Traditional breeding methods, when applicable, can subsequently be used to incorporate the entire pathway into a single plant. Successful production of the PHA polyhydroxybutyrate in cells of *Arabidopsis* has been demonstrated by Poirier, Y. et al. (1992, *Science* 256:520-523), and in plastids thereof by Nawrath et al. (1994, *Proc. Natl. Acad. Sci. USA* 91:12760-12764).

Specific methods for transforming a wide variety of dicots and obtaining transgenic plants are well documented in the literature (see Gasser, C.S. and Fraley, R.T. (1989) *Science* 244:1293-1299; Fisk, H.J. and Dandekar, A.M. (1993) *Scientia Horticulturae* 55:5-36; Christou (1994) *Agro Food Industry Hi Tech* (March/April 1994) p. 17, and the references cited therein).

Successful transformation and plant regeneration have been achieved in the monocots as follows: asparagus (*Asparagus officinalis*; Bytebier et al. (1987) *Proc. Natl. Acad. Sci. USA* 84:5345); barley (*Hordeum vulgarae*; Wan, Y. and Lemaux, P.G. (1994) *Plant Physiol.* 104:37-48); maize (*Zea mays*; Rhodes, C.A. et al. (1988) *Science* 240:204-207; Gordon-Kamm, W.J. et al. (1990) *Plant Cell* 2:603-618; Fromm, M.E. et al. (1990) *Bi*/*Technology* 8:833-839; Koziel et al. (1993) *Bio*/*Technology* 11:194); oats (*Avena sativa*; Somers et al. (1992) *Bio*/*Technology* 10:1589); orchardgrass (*Dactylis glomerata*; Horn, M.E. et al. (1988) *Plant Cell Rep.* 7:469-472); rice (*Oryza sativa*, including indica and japonica varieties; Toriyama, K. et al. (1988) *Bio*/*Technology* 6:1072-1074; Zhang, H.M. et al. (1988) *Plant Cell Rep.* 7:379-384; Luo and Wu (1988) *Plant Mol. Biol. Rep.* 6:165; Zhang, W. and Wu, R. (1988) *Theor. Appl. Genet.* 76:835-840; Christou et al. (1991) *Bio*/*Technology* 9:957); rye (*Secale cereale;* De la Pena et al. (1987) *Nature* 325:274); sorghum (*Sorghum bicolor;* Casas, A.M. et al. (1993) *Proc. Natl. Acad Sci. USA* 90:11212-11216); sugar cane (*Saccharum* spp.; Bower and Birch (1992) *Plant J*. 2:409); tall fescue (*Festuca arundinacea*; Wang, Z.Y. et al. (1992) *Bio*/*Technology* 10:691-696); turfgrass (*Agrostis palustris*; Zhong, H. et al. (1993) *Plant Cell Rep.* 13:1-6); and wheat (*Triticum aestivum*; Vasil et al. (1992) *Bio*/*Technology* 10:667; Weeks, J.T. et al. (1993) *Plant Physiol*. 102:1077-1084; Becker, D. et al. (1994) *Plant J*. 5:299-307).

Particularly useful plants for polyester production include those, such as potato and sugarbeet, that produce carbon substrates which can be employed for polyester biosynthesis. Cereal plants such as com, wheat, and rice are also preferred. Other useful plants include tobacco and high oil seed plants such as soybean, canola, oil seed rape, *Arabidopsis sp.* and peanut. Plants that grow in desert or in mineralized soil can also be employed for the production of polyesters. Polymers that can be produced in this manner include but are not limited to for example, polyhydroxybutyrate, and copolymers incorporating both short chain length and medium chain length monomers, such as polyhydroxybutyrate -co- polyhydroxycaproate, polyhydroxycaproate -co- polyhydroxyoctanoate, and polyhydroxyoctanoate -co- polyhydroxydecanoate.

All of the compositions and/or methods disclosed and claimed herein can be made and executed without undue experimentation in light of the present disclosure.

## Claims

1. An isolated nucleic acid segment comprising recombinant genes encoding:
a) a polyhydroxyalkanoic acid synthase protein; and
b) a fatty acid:acyl-CoA transferase protein; said genes being heterologous within the nucleic acid segment

2. The nucleic acid segment of claim 1, wherein the polyhydroxyalkanoic acid synthase protein is an *Alcaligenes eutrophus* polyhydroxyalkanoic acid synthase protein.

3. The nucleic acid segment of claim 2, wherein the *Alcaligenes eutrophus* polyhydroxyalkanoic acid synthase protein is encoded by the *Alcaligenes eutrophus phaC* polyhydroxyalkanoic acid synthase structural gene.

4. The nucleic acid segment of claim 1, wherein the fatty acid:acyl-CoA transferase protein is a 4-hydroxyburyrate:acyl-CoA transferase protein.

5. The nucleic acid segment of claim 4, wherein the 4-hydroxybutyrate:acyl-CoA transferase protein is a *Clostridium kluyveri* 4-hydroxybutyrate:acyl-CoA transferase protein.

6. The nucleic acid segment of claim 5, wherein the *Clostridium kluyveri* 4-hydroxybutyrate:acyl-CoA transferase protein is encoded by the *Clostridium kluyveri orfZ* 4-hydroxybutyrate:acyl-CoA transferase structural gene.

7. The nucleic acid segment of claim 1, further comprising a promoter functional in bacterial cells.

8. The nucleic acid segment of claim 1, wherein:
a) the sequence encoding a polyhydroxyalkanoic acid synthase protein is operably linked to its native promoter, and
b) the sequence encoding a fatty acid:acyl-CoA transferase protein is operably linked to its native promoter.

9. The nucleic acid segment of claim 1, further comprising a promoter heterologous to:
a) the sequence encoding a polyhydroxyalkanoic acid synthase protein; and
b) the sequence encoding a fatty acid:acyl-CoA transferase protein.

10. A recombinant vector comprising the nucleic acid segment of claim 1.

11. A recombinant vector comprising the nucleic acid segment of claim 9.

12. The recombinant vector of claim 11 having accession number DSM 11427.

13. The recombinant vector of claim 11 having accession number DSM 11435

14. A recombinant vector comprising:
a) an isolated nucleic acid segment encoding a polyhydroxyalkanoic acid synthase protein:
b) an isolated nucleic acid segment encoding a fatty acid:acyl-CoA transferase protein: and
c) promoter heterologous to the nucleic acid segment encoding a polyhydroxyalkanoic acid synthase protein, and heterologous to the nucleic acid segment encoding a fatty acid:acyl-CoA transferase protein.

15. A cell expressing:
a) a polyhydroxyalkanoic acid synthase protein; and
b) a fatty acid:acyl-CoA transferase protein; wherein each said protein is encoded by a recombinant gene located or a DNA segment within which the gene is heretologous

16. The cell of claim 15, further defined as a plant cell, mammalian cell, insect cell, fungal cell, or bacterial cell.

17. The cell of claim 16, wherein the cell is a plant cell.

18. The cell of claim 16, wherein the cell is a bacterial cell.

19. The bacterial cell of claim 18, wherein the bacterial cell is *Escherichia coli*.

20. The bacterial cell of claim 19, wherein the bacterial cell is *Escherichia coli* strain XL1-Blue.

21. The cell of claim 15, wherein the polyhydroxyalkanoic acid synthase protein is a *Alcaligenes eutrophus* polyhydroxyalkanoic acid synthase protein.

22. The cell of claim 21, wherein the *Alcaligenes eutrophus* polyhydroxyalkanoic acid synthase protein is encoded by the *Alcaligenes eutrophus phaC* polyhydroxyalkanoic acid synthase structural gene.

23. The cell of claim 15, wherein the fatty acid:acyl-CoA transferase protein is a 4-hydroaybutyrate:acyl-CoA transferase protein.

24. The cell of claim 23, wherein the 4-hydroxybutyrate:acyl-CoA transferase protein is a *Clostridium kluyveri* 4-hydroxybutyrate:acyl-CoA transferase protein.

25. The cell of claim 24, wherein the *Clostridium kluyveri* 4-hydroxybutyrate:acyl-CoA transferase protein is encoded by the *Clostridium kluyveri orfZ* 4-hydroxybutyrate:acyl-CoA transferase structural gene.

26. A method for preparing a transformed cell, comprising the steps:
a) selecting a host cell:
b) contacting the host cell and an isolated nucleic acid segment, the nucleic acid segment encoding:
i) a polyhydroxyalkanoic acid synthase protein; and
ii) a fatty acid:acyl-CoA transferase protein;
under conditions suitable for uptake of the nucleic acid segment by the host cell; and
c) regenerating the cell to produce a transformed cell;
wherein the nucleic acid segment is free of total genomic DNA

27. The method of claim 26, wherein the contacting step is further defined as calcium chloride mediated transformation.

28. The method of claim 26, wherein the cell is a plant cell, mammalian cell, insect cell, fungal cell, or bacterial cell.

29. The method of claim 28, wherein the cell is a plant cell.

30. The method of claim 28, wherein the cell is a bacterial cell.

31. The method of claim 30, wherein the bacterial cell is *Escherichia coli*.

32. The method of claim 31, wherein the bacterial cell is *Escherichia coli* strain XL 1-Blue.

33. The method of claim 26, wherein the polyhydroxyalkanoic acid synthase protein is an *Alcaligenes eutrophus* polyhydroxyalkanoic acid synthase protein.

34. The method of claim 33, wherein the *Alcaligenes eutrophus* polyhydroxyalkanoic acid synthase protein is encoded by the *Alcaligenes eutrophus phaC* polyhydroxyalkanoic acid synthase structural gene.

35. The method of claim 26, wherein the fatty acid:acyl-CoA transferase protein is a 4-hydroxybutyrate:acyl-CoA transferase protein.

36. The method of claim 35, wherein the 4-hydroxybutyrate:acyl-CoA transferase protein is a *Clostridium kluyveri* 4-hydroxybutyrate:acyl-CoA transferase protein.

37. The method of claim 36, wherein the *Clostridium kluyveri* 4-hydroxybutyrate:acyl-CoA transferase protein is encoded by the *Clostridium kluyveri orfZ* 4-hydroxybutyrate:acyl-CoA transferase structural gene.

38. A method for the preparation of a polyester, comprising the steps of:
a) obtaining a cell capable of producing:
i) a polyhydroxyalkanoic acid synthase protein; and
ii) a fatty acid:acyl-CoA transferase protein; wherein each said protein is encoded by a recombinant gene located or a DNA segment within which the gene is heterologous;
b) establishing a culture of the cell;
c) culturing the cell under conditions suitable for the production of the polyester; and
d) isolating the polyester from the cell.

39. The method of claim 38, wherein the cell is a plant cell, mammalian cell, insect cell, fungal cell, or bacterial cell.

40. The method of claim 39, wherein the cell is a plant cell.

41. The method of claim 39, wherein the cell is a bacterial cell.

42. The method of claim 41, wherein the bacterial cell is *Escherichia coli*.

43. The method of claim 42, wherein the bacterial cell is *Escherichia coli* strain XL1-Blue.

44. The method of claim 38. wherein the polyhydroxyalkanoic acid synthase protein is a polyhydroxyalkanoic acid synthase protein from *Alcaligenes eutrophus*.

45. The method of claim 44, wherein the *Alcaligenes eutrophus* polyhydroxyalkanoic acid synthase protein is encoded by the *Alcaligenes eutrophus phaC* polyhydroxyalkanoic acid synthase structural gene.

46. The method of claim 38, wherein the fatty acid:acyl-CoA transferase protein is a 4-hydroxybutyrate:acyl-CoA transferase protein.

47. The method of claim 46, wherein the 4-hydroxybutyrate:acyl-CoA transferase protein is a *Clostridium kluyveri* 4-hydroxybutyrate:acyl-CoA transferase protein.

48. The method of claim 47, wherein the *Clostridium kluyveri* 4-hydroxybutyrate:acyl-CoA transferase protein is encoded by the *Clostridium kluyveri* ortZ 4-hydroxybutyrate:acyl-CoA transferase structural gene.

49. The method of claim 38, wherein the culture contains glucose.

50. The method of claim 33, wherein the culture contains 4-hydroxybutyric acid, the sodium salt of 4-hydroxybutyric acid, γ-butyrolactone, 1,4-butanediol, 4-hydroxyvaleric acid, γ-valerolactone, 1,4-pentanediol, 3-hydroxybutyric acid, the sodium salt of 3-hydroxybutyric acid, a hydroxypropionic acid, a hydroxybutyric acid, a hydroxyvaleric acid, a hydroxycaproic acid, a hydroxyheptanoic acid, a hydroxyoctanoic acid, a hydroxydecanoic acid, γ-caprolactone, γ-heptanoloactone, γ-octanolactone, or γ-decanolactone.

51. The method of claim 38, wherein the culture contains molecular oxygen.

52. The method of claim 38, wherein the cell is further capable of producing a protein capable of hydrolysing a lactone to the corresponding hydroxyalkanoic acid.

53. The method of claim 38, wherein the cell is further capable of producing a 2-oxoglutarate decarboxylase protein and a 4-hydroxybutyrate dehydrogenase protein.

54. The method of claim 38, wherein the cell is further capable of producing a succinate:acetyl-CoA transferase protein, a succinate-semialdehyde dehydrogenase protein, and a 4-hydroxybutyrate dehydrogenase protein.

55. The method of claim 38, wherein the cell is further capable of producing a succinate-semialdehyde dehydrogenase protein, and a 4-hydroxybutyrate dehydrogenase protein.

56. The method of claim 38, wherein the cell is further capable of producing a 2-methylcitrate synthase protein, a 2-methylcitrate dehydratase protein, a 2-methylisocitrate dehydratase protein, a 2-methylisocitrate lyase protein, a succinate:acetyl-CoA transferase protein, a succinate-semialdehyde dehydrogenase protein, and a 4-hydroxybutyrate dehydrogenase protein.

57. The method of claim 38, wherein the polyester is a homopolyester.

58. The method of claim 57, wherein the homopolyester is poly(4-hydroxybutyric acid).

59. The method of claim 57, wherein the homopolyester is poly(3-hydroxybutyric acid).

60. The method of claim 38, wherein the polyester is a copolyester.

61. The method of claim 60, wherein the copolyester is poly(3-hydroxybutyric acid -co- 4-hydroxybutyric acid).

62. An isolated nucleic acid segment, a recombinant vector or a cell according to any one of Claims 1 to 25, further comprising a recombinant gene encoding β-ketothiolase.

## Patentansprüche

1. Isolierter Nucleinsäureabschnitt, der rekombinante Gene aufweist, die codieren für
a) ein Polyhydroxyfettsäure-Synthase-Protein, und
b) ein Fettsäure:Acyl-CoA-Transferase-Protein,
wobei die genannten Gene bezüglich des Nucleinsäureabschnitts heterolog sind.

2. Nucleinsäureabschnitt nach Anspruch 1, wobei das Polyhydroxyfettsäure-Synthase-Protein ein Polyhydroxyfettsäure-Synthase-Protein von *Alcaligenes eutrophus* ist

3. Nucleinsäureabschnitt nach Anspruch 2, wobei das Polyhydroxyfettsäure-Synthase-Protein von *Alcaligenes eutrophus* durch das *phaC*-Strukturgen der Polyhydroxyfettsäure-Synthase von *Alcaligenes eutrophus* codiert wird.

4. Nucleinsäureabschnitt nach Anspruch 1, wobei das Fettsäure:Acyl-CoA-Transferase-Protein ein 4-HydroxybutyratAcyl-CoA-Transferase-Protein ist.

5. Nucleinsäureabschnitt nach Anspruch 4, wobei das 4-Hydroxybutyrat:Acyl-CoA-Transferase-Protein ein 4-Hydroxybutyrat:Acyl-CoA-Transferase-Protein von *Clostridium kluyveri* ist.

6. Nucleinsäureabschnitt nach Anspruch 5, wobei das 4-Hydroxybutyrat:Acyl-CoA-Transferase-Protein von *Clostridium kluyveri* durch das *orfZ*-Strukturgen der 4-Hydroxybutyrat:Acyl-CoA-Transferase von *Clostridium kluyveri* codiert wird.

7. Nucleinsäureabschnitt nach Anspruch 1, wobei der Abschnitt ferner einen Promotor umfasst, der in Bakterienzellen funktionell ist.

8. Nucleinsäureabschnitt nach Anspruch 1, wobei:
a) die Sequenz, die für ein Polyhydroxyfettsäure-Synthase-Protein codiert, funktionsfähig mit ihrem nativen Promotor verknüpft ist, und
b) die Sequenz, die für ein Fettsäure:Acyl-CoA-Transferase-Protein codiert funktionsfähig mit ihrem nativen Promotor verknüpft ist.

9. Nucleinsäureabschnitt nach Anspruch 1, wobei der Abschnitt ferner einen Promotor umfasst, der heterolog ist bezüglich:
a) der Sequenz, die für ein Polyhydroxyfettsäure-Synthase-Protein codiert, und
b) der Sequenz, die für ein Fettsäure:Acyl-CoA-Transferase-Protein codiert.

10. Rekombinanter Vektor, der den Nucleinsäureabschnitt nach Anspruch 1 aufweist.

11. Rekombinanter Vektor, der den Nucleinsäureabschnitt nach Anspruch 9 aufweist.

12. Rekombinanter Vektor nach Anspruch 11, wobei der Vektor die Zugangsnummer DSM 11472 hat.

13. Rekombinanter Vektor nach Anspruch 11, wobei der Vektor die Zugangsnummer DSM 11435 hat.

14. Rekombinanter Vektor, der umfasst:
a) einen isolierten Nucleinsäureabschnitt, der für ein Polyhydroxyfettsäure-Synthase-Protein codiert,
b) einen isolierten Nucleinsäureabschnitt, der für ein Fettsäure:Acyl-CoA-Transferase-Protein codiert, und
c) einen Promotor, der heterolog bezüglich des Nucleinsäureabschnitts ist, der für ein Polyhydroxyfettsäure-Synthase-Protein codiert, und heterolog bezüglich des Nucleinsäureabschnitts, der für ein Fettsäure:Acyl-CoA-Transferase-Protein codiert.

15. Zelle, die exprimiert:
a) ein Polyhydroxyfettsäure-Synthase-Protein, und
b) ein Fettsäure:Acyl-CoA-Transferase-Protein,
wobei jedes der genannten Proteine durch ein rekombinantes Gen codiert wird, das auf einem DNA-Abschnitt liegt, bezüglich dessen das Gen heterolog ist.

16. Zelle nach Anspruch 15, ferner definiert als eine Pflanzenzelle, Säugerzelle, Insektenzelle, Pilzzelle oder Bakterienzelle.

17. Zelle nach Anspruch 16, wobei die Zelle eine Pflanzenzelle ist.

18. Zelle nach Anspruch 16, wobei die Zelle eine Bakterienzelle ist.

19. Bakterienzelle nach Anspruch 18, wobei die Bakterienzelle *Escherichia coli* ist.

20. Bakterienzelle nach Anspruch 19, wobei die Bakterienzelle *Escheriehia coli*, Stamm XL1-Blue, ist.

21. Zelle nach Anspruch 15, wobei das Polyhydroxyfettsäure-Synthase-Protein ein Polyhydroxyfettsäure-Synthase-Protein von *Alcaligenes eutrophus* ist.

22. Zelle nach Anspruch 21, wobei das Polyhydroxyfettsäure-Synthase-Protein von *Alcaligenes eutrophus* durch das *phaC*-Strukturgen der Polyhydroxyfettsäure-Synthase von *Alcaligenes eutrophus* codiert wird.

23. Zelle nach Anspruch 15, wobei das Fettsäure:Acyl-CoA-Transferase-Protein ein 4-Hydroxybutyrat.Acyl-CoA-Transferase-Protein ist.

24. Zelle nach Anspruch 23, wobei das 4-Hydroxybutyrat:Acyl-CoA-Transferase-Protein ein 4-Hydroxybutyrat:Acyl-CoA-Transferase-Protein von *Clostridium kluyveri* ist.

25. Zelle nach Anspruch 24, wobei das 4-Hydroxybutyrat:Acyl-CoA-Transferase-Protein von *Clostridium kluyveri* durch das *orfZ*-Strukturgen der 4-Hydroxybutyrat:Acyl-CoA-Transferase von *Clostridium kluyveri* codiert wird.

26. Verfahren zur Herstellung einer transformierten Zelle, das die Schritte umfasst:
a) Auswählen einer Wirtszelle,
b) Inkontaktbringen der Wirtszelle mit einem isolierten Nucleinsäureabschnitt, wobei der Nucleinsäureabschnitt codiert für:
i) ein Polyhydroxyfettsäure-Synthase-Protein, und
ii) ein Fettsäure:Acyl-CoA-Transferase-Protein,
unter Bedingungen, die für die Aufnahme des Nucleinsätireabschnitts durch die Wirtszelle geeignet sind, und
c) Regenerieren der Zelle zur Erzeugung einer transformierten Zelle,
wobei der Nucleinsäureabschnitt frei von gesamter genomischer DNA ist.

27. Verfahren nach Anspruch 26, wobei der Schritt des Inkontaktbringens ferner als eine Calciumchlorid-vermittelte Transformation definiert ist.

28. Verfahren nach Anspruch 26, wobei die Zelle eine Pflanzenzelle, Säugerzelle, Insektenzelle, Pilzzelle oder Bakterienzelle ist.

29. Verfahren nach Anspruch 28, wobei die Zelle eine Pflanzenzelle ist.

30. Verfahren nach Anspruch 28, wobei die Zelle eine Bakterienzelle ist.

31. Verfahren nach Anspruch 30, wobei die Bakterienzelle *Escherichia coli* ist.

32. Verfahren nach Anspruch 31, wobei die Bakterienzelle *Escherichia coli,* Stamm XL1-Blue, ist.

33. Verfahren nach Anspruch 26, wobei das Polyhydroxyfettsäure-Synthase-Protein ein Polyhydroxyfettsäure-Synthase-Protein von *Alcaligenes eutrophus* ist.

34. Verfahren nach Anspruch 33, wobei das Polyhydroxyfettsäure-Synthase-Protein von *Alcaligenes eutrophus* durch das *phaC*-Strukturgen der Potyhydroxyfettsäure-Synthase von *Alcaligenes eutrophus* codiert wird.

35. Verfahren nach Anspruch 26, wobei das Fettsäure:Acyl-CoA-Transferase-Protein ein 4-Hydroxybutyrat:Acyl-CoA-Transferase-Protein ist.

36. Verfahren nach Anspruch 35, wobei das 4-Hydroxybutyrat:Acyl-CoA-Transferase-Protein ein 4-Hydroxybutyrat:Acyl-CoA-Transferase-Protein von *Clostridium kluyveri* ist.

37. Verfahren nach Anspruch 36, wobei das 4-Hydroxybutyrat:Acyl-CoA-Transferase-Protein von *Clostridium kluyveri* durch das *orfZ*-Strukturgen der 4-Hydroxybutyrat:Acyl-CoA-Transferase von *Clostridium kluyveri* codiert wird.

38. Verfahren zur Herstellung eines Polyesters, das die Schritte umfasst:
a) Gewinnen einer Zelle, die imstande ist zu erzeugen:
i) ein Polyhydroxyfettsäure-Synthase-Protein, und
ii) ein Fettsäure:Acyl-CoA-Transferase-Protein,
wobei jedes der genannten Proteine durch ein rekombinantes Gen codiert wird, das auf einem DNA-Abschnitt liegt, bezüglich dessen das Gen heterolog ist,
b) Etablieren einer Kultur der Zelle,
c) Kultivieren der Zelle unter Bedingungen, die für die Erzeugung des Polyesters geeignet sind, und
d) Isolieren des Polyesters aus der Zelle.

39. Verfahren nach Anspruch 38, wobei die Zelle eine Pflanzenzelle, Säugerzelle, Insektenzelle, Pilzzelle oder Bakterienzelle ist.

40. Verfahren nach Anspruch 39, wobei die Zelle eine Pflanzenzelle ist.

41. Verfahren nach Anspruch 39, wobei die Zelle eine Bakterienzelle ist.

42. Verfahren nach Anspruch 41, wobei die Bakterienzelle *Escherichia coli* ist.

43. Verfahren nach Anspruch 42, wobei die Bakterienzelle *Escherichia coli*, Stamm XL1-Blue, ist.

44. Verfahren nach Anspruch 38, wobei das Polyhydroxyfettsäure-Synthase-Protein ein Polyhydroxyfettsäure-Synthase-Protein von *Alcaligenes eutrophus* ist.

45. Verfahren nach Anspruch 44, wobei das Polyhydroxyfettsäure-Synthase-Protein von *Alcaligenes eutrophus* durch das *phaC*-Strukturgen der Polyhydroxyfettsäure-Synthase von *Alcaligenes eutrophus* codiert wird.

46. Verfahren nach Anspruch 38, wobei das Fettsäure:Acyl-CoA-Transferase-Protein ein 4-Hydroxybutyrat:Acyl-CoA-Transferase-Protein ist.

47. Verfahren nach Anspruch 46, wobei das 4-Hydroxybutyrat:Acyl-CoA-Transferase-Protein ein 4-Hydroxybutyrat:Acyl-CoA-Transferase-Protein von *Clostridium kluyveri* ist.

48. Verfahren nach Anspruch 47, wobei das 4-Hydroxybutyrat:Acyl-CoA-Transferase-Protein von *Clostridium kluyveri* durch das *orfZ*-Strukturgen der 4-Hydroxybutyrat:Acyl-CoA-Transferase von *Clostridium kluyveri* codiert wird.

49. Verfahren nach Anspruch 38, wobei die Kultur Glucose enthält.

50. Verfahren nach Anspruch 38, wobei die Kultur enthält 4-Hydroxybuttersäure, das Natriumsalz der 4-Hydroxybuttersäure, γ-Butyrolacton, 1,4-Butandiol, 4-Hydroxyvaleriansäure, γ-Valerolacton, 1,4-Pentandiol, 3-Hydroxybuttersäure, das Natriumsalz der 3-Hydroxybuttersäure, eine Hydroxypropionsäure, eine Hydroxybuttersäure, eine Hydroxyvaleriansäure, eine Hydroxycapronsäure, eine Hydroxyheptansäure, eine Hydroxyoctansäure, eine Hydroxydecansäure, γ-Caprolacton, γ-Heptalacton, γ-Octalacton oder γ-Decalacton.

51. Verfahren nach Anspruch 38, wobei die Kultur molekularen Sauerstoff enthält.

52. Verfahren nach Anspruch 38, wobei die Zelle ferner imstande ist, ein Protein zu erzeugen, das imstande ist, ein Lacton zur entsprechenden Hydroxyfettsäure zu hydrolysieren.

53. Verfahren nach Anspruch 38, wobei die Zelle ferner imstande ist, ein 2-Oxoglutarat-Decarboxylase-Protein und ein 4-Hydroxybutyrat-Dehydrogenase-Protein zu erzeugen.

54. Verfahren nach Anspruch 38, wobei die Zelle ferner imstande ist, ein Succinat:Acetyl-CoA-Transferase-Protein, ein Succinatsemialdehyd-Dehydrogenase-Protein und ein 4-Hydroxybutyrat-Dehydrogenase-Protein zu erzeugen.

55. Verfahren nach Anspruch 38, wobei die Zelle ferner imstande ist, ein Succinatsemialdehyd-Dehydrogenase-Protein und ein 4-Hydroxybutyrat-Dehydrogenase-Protein zu erzeugen.

56. Verfahren nach Anspruch 38, wobei die Zelle ferner imstande ist, ein 2-Methylcitrat-Synthase-Protein, ein 2-Methylcitrat-Dehydratase-Protein, ein 2-Methylisocitrat-Dehydratase-Protein, ein 2-Methylisocitrat-Lyase-Protein, ein Succinat:Acetyl-CoA-Transferase-Protein, ein Succinatsemialdehyd-Dehydrogenase-Protein und ein 4-Hydroxybutyrat-Dehydrogenase-Protein zu erzeugen.

57. Verfahren nach Anspruch 38, wobei der Polyester ein Homopolyester ist.

58. Verfahren nach Anspruch 57, wobei der Homopolyester Poly(4-hydroxybuttersäure) ist.

59. Verfahren nach Anspruch 57, wobei der Homopolyester Poly(3-hydroxybuttersäure) ist.

60. Verfahren nach Anspruch 38, wobei der Polyester ein Copolyester ist.

61. Verfahren nach Anspruch 60, wobei der Copolyester Poly(3-hydroxybuttersäure-co-4-hydroxybuttersäure) ist.

62. Isolierter Nucleinsäureabschnitt, rekombinanter Vektor oder Zelle gemäß einem beliebiger der Ansprüche 1 bis 25, ferner umfassend ein rekombinantes Gen, das für die β-Ketothiolase codiert.

## Revendications

1. Segment d'acide nucléique isolé comprenant des gènes recombinants codant :
a) une protéine acide polyhydroxyalcanoïque synthase ; et
b) une protéine acide gras:acyl-CoA transférase ;
lesdits gènes étant hétérologues dans le segment d'acide nucléique.

2. Segment d'acide nucléique selon la revendication 1, dans lequel la protéine acide polyhydroxyalcanoïque synthase est une protéine acide polyhydroxyalcanoïque synthase de *Alcaligenes eutrophus*.

3. Segment d'acide nucléique selon la revendication 2, dans lequel la protéine acide polyhydroxyalcanoïque synthase de *Alcaligenes eutrophus* est codée par le gène structural d'acide polyhydroxyalcanoïque synthase de *Alcaligenes eutrophus phaC*.

4. Segment d'acide nucléique selon la revendication 1, dans lequel la protéine acide gras:acyl-CoA transférase est une protéine 4-hydroxybutyrate:acyl-CoA transférase.

5. Segment d'acide nucléique selon la revendication 4, dans lequel la protéine 4-hydroxybutyrate:acyl-CoA transférase est une protéine 4-hydroxybutyrate:acyl-CoA transférase de *Clostridium kluyveri*.

6. Segment d'acide nucléique selon la revendication 5, dans lequel la protéine 4-hydroxybutyrate:acyl-CoA transférase de *Clostridium kluyveri* est codée par le gène structural de 4-hydroxybutyrate:acyl-CoA transférase de *Clostridium kluyveri orfZ*.

7. Segment d'acide nucléique selon la revendication 1, comprenant en outre un promoteur fonctionnel dans des cellules bactériennes.

8. Segment d'acide nucléique selon la revendication 1, dans lequel :
a) la séquence codant une protéine acide polyhydroxyalcanoïque synthase est liée de façon opératoire à son promoteur natif ; et
b) la séquence codant une protéine acide gras:acyl-CoA transférase est liée de façon opératoire à son promoteur natif.

9. Segment d'acide nucléique selon la revendication 1, comprenant en outre un promoteur hétérologue à :
a) la séquence codant une protéine acide polyhydroxyalcanoïque synthase ; et
b) la séquence codant une protéine acide gras:acyl-CoA transférase.

10. Vecteur recombinant comprenant le segment d'acide nucléique selon la revendication 1.

11. Vecteur recombinant comprenant le segment d'acide nucléique selon la revendication 9.

12. Vecteur recombinant selon la revendication 11 ayant le numéro d'ordre DSM 11427.

13. Vecteur recombinant selon la revendication 11, ayant le numéro d'ordre DSM 11435.

14. Vecteur recombinant comprenant :
a) un segment d'acide nucléique isolé codant une protéine acide polyhydroxyalcanoïque synthase ;
b) un segment d'acide nucléique isolé codant une protéine acide gras:acyl-CoA transférase ; et
c) un promoteur hétérologue au segment d'acide nucléique codant une protéine acide polyhydroxyalcanoïque synthase, et hétérologue au segment d'acide nucléique codant une protéine acide gras:acyl-CoA transférase.

15. Cellule exprimant :
a) une protéine acide polyhydroxyalcanoïque synthase ; et
b) une protéine acide gras:acyl-CoA transférase ;
dans laquelle chaque dite protéine est codée par un gène recombinant situé sur un segment d'ADN dans lequel le gène est hétérologue.

16. Cellule selon la revendication 15, définie en outre comme étant une cellule de plante, une cellule mammifère, une cellule d'insecte, une cellule fongique ou une cellule bactérienne.

17. Cellule selon la revendication 16, la cellule étant une cellule de plante.

18. Cellule selon la revendication 16, la cellule étant une cellule bactérienne.

19. Cellule bactérienne selon la revendication 18, la cellule bactérienne étant *Escherichia coli*.

20. Cellule bactérienne selon la revendication 19, la cellule bactérienne étant la souche XL1-Blue de *Escherichia coli*.

21. Cellule selon la revendication 15, dans laquelle la protéine acide polyhydroxyalcanoïque synthase est une protéine acide polyhydroxyalcanoïque synthase de *Alcaligenes eutrophus*.

22. Cellule selon la revendication 21, dans laquelle la protéine acide polyhydroxyalcanoïque synthase de *Alcaligenes eutrophus* est codée par le gène structural d'acide polyhydroxyalcanoïque synthase de *Alcaligenes eutrophus phaC*.

23. Cellule selon la revendication 15, dans laquelle la protéine acide gras:acyl-CoA transférase est une protéine 4-hydroxybutyrate:acyl-CoA transférase.

24. Cellule selon la revendication 23, dans laquelle la protéine 4-hydroxybutyrate:acyl-CoA transférase est une protéine 4-hydroxybutyrate:acyl-CoA transférase de *Clostridium kluyveri*.

25. Cellule selon la revendication 24, dans laquelle la protéine 4-hydroxybutyrate:acyl-CoA transférase de *Clostridium kluyveri* est codée par le gène structural de 4-hydroxybutyrate:acyl-CoA transférase de *Clostridium kluyveri orfZ*.

26. Procédé pour préparer une cellule transformée, comprenant les étapes consistant à :
a) choisir une cellule hôte ;
b) mettre en contact la cellule hôte et un segment d'acide nucléique isolé, le segment d'acide nucléique codant :
i) une protéine acide polyhydroxyalcanoïque synthase ; et
ii) une protéine acide gras:acyl-CoA transférase ;
dans des conditions appropriées pour l'absorption du segment d'acide nucléique par la cellule hôte ; et
c) la régénération de la cellule pour produire une cellule transformée ;
dans lequel le segment d'acide nucléique est exempt d'ADN génomique total.

27. Procédé selon la revendication 26, dans lequel l'étape de mise en contact est en outre définie comme étant une transformation due à du chlorure de calcium.

28. Procédé selon la revendication 26, dans lequel la cellule est une cellule de plante, une cellule mammifère, une cellule d'insecte, une cellule fongique ou une cellule bactérienne.

29. Procédé selon la revendication 28, dans lequel la cellule est une cellule de plante.

30. Procédé selon la revendication 28, dans lequel la cellule est une cellule bactérienne.

31. Procédé selon la revendication 30, dans lequel la cellule bactérienne est *Escherichia coli*.

32. Procédé selon la revendication 31, dans lequel la cellule bactérienne est la souche XL1-Blue de *Escherichia coli*.

33. Procédé selon la revendication 26, dans lequel la protéine acide polyhydroxyalcanoïque synthase est une protéine acide polyhydroxyalcanoïque synthase de *Alcaligenes eutrophus.*

34. Procédé selon la revendication 33, dans lequel la protéine acide polyhydroxyalcanoïque synthase de *Alcaligenes eutrophus* est codée par le gène structural d'acide polyhydroxyalcanoïque synthase d'*Alcaligenes eutrophus phaC*.

35. Procédé selon la revendication 26, dans lequel la protéine acide gras:acyl-CoA transférase est une protéine 4-hydroxybutyrate:acyl-CoA transférase.

36. Procédé selon la revendication 35, dans lequel la protéine 4-hydroxybutyrate:acyl-CoA transférase est une protéine 4-hydroxybutyrate:acyl-CoA transférase de *Clostridium kluyveri*.

37. Procédé selon la revendication 36, dans lequel la protéine 4-hydroxybutyrate:acyl-CoA transférase de *Clostridium kluyveri* est codée par le gène structural de 4-hydroxybutyrate:acyl-CoA transférase de *Clostridium kluyveri orfZ*.

38. Procédé pour la préparation d'un polyester, comprenant les étapes consistant à :
a) obtenir une cellule apte à produire :
i) une protéine acide polyhydroxyalcanoïque synthase ; et
ii) une protéine acide gras:acyl-CoA transférase ;
où chaque dite protéine est codée par un gène recombinant situé sur un segment d'ADN dans lequel le gène est hétérologue ;
b) mettre la cellule en culture dans des conditions appropriées pour la production du polyester ; et
d) isoler le polyester de la cellule.

39. Procédé selon la revendication 38, dans lequel la cellule est une cellule de plante, une cellule mammifère, une cellule d'insecte, une cellule fongique ou une cellule bactérienne.

40. Procédé selon la revendication 39, dans lequel la cellule est une cellule de plante.

41. Procédé selon la revendication 39, dans lequel la cellule est une cellule bactérienne.

42. Procédé selon la revendication 41, dans lequel la cellule bactérienne est *Escherichia coli*.

43. Procédé selon la revendication 42, dans lequel la cellule bactérienne est la souche XL1-Blue de *Escherichia coli*.

44. Procédé selon la revendication 38, dans lequel la protéine acide polyhydroxyalcanoïque synthase est une protéine acide polyhydroxyalcanoïque synthase de *Alcaligenes eutrophus*.

45. Procédé selon la revendication 44, dans lequel la protéine acide polyhydroxyalcanoïque synthase de *Alcaligenes eutrophus* est codée par le gène structural d'acide polyhydroxyalcanoïque synthase de *Alcaligenes eutrophus phaC*.

46. Procédé selon la revendication 38, dans lequel la protéine acide gras:acyl-CoA transférase est une protéine 4-hydroxybutyrate:acyl-CoA transférase.

47. Procédé selon la revendication 46, dans lequel la protéine 4-hydroxybutyrate:acyl-CoA transférase est une protéine 4-hydroxybutyrate:acyl-CoA transférase de *Clostridium kluyveri*.

48. Procédé selon la revendication 47, dans lequel la protéine 4-hydroxybutyrate:acyl-CoA transférase de *Clostridium kluyveri* est codée par le gène structural de 4-hydroxybutyrate:acyl-CoA transférase de *Clostridium kluyveri orfZ*.

49. Procédé selon la revendication 38, dans lequel la culture contient du glucose.

50. Procédé selon la revendication 38, dans lequel la culture contient de l'acide 4-hydroxybutyrique, le sel sodique d'acide 4-hydroxybutyrique, de la γ-butyrolactone, du 1,4-butanediol, de l'acide 4-hydroxyvalérique, de la γ-valérolactone, du 1,4-pentanediol, de l'acide 3-hydroxybutyrique, le sel sodique d'acide 3-hydroxybutyrique, un acide hydroxypropionique, un acide hydroxybutyrique, un acide hydroxyvalérique, un acide hydroxycaproïque, un acide hydroxyheptanoïque, un acide hydroxyoctanoïque, un acide hydroxydécanoïque, de la γ-caprolactone, de la γ-heptanoloactone, de la γ-octanolactone ou de la γ-décanolactone.

51. Procédé selon la revendication 38, dans lequel la culture contient de l'oxygène moléculaire.

52. Procédé selon la revendication 38, dans lequel la cellule est en outre apte à produire une protéine capable d'hydrolyser une lactone en l'acide hydroxy-alcanoïque correspondant.

53. Procédé selon la revendication 38, dans lequel la cellule est en outre apte à produire une protéine 2-oxoglutarate décarboxylase et une protéine 4-hydroxybutyrate déshydrogénase.

54. Procédé selon la revendication 38, dans lequel la cellule est en outre apte à produire une protéine succinate:acétyl-CoA transférase, une protéine succinate-semialdéhyde déshydrogénase et une protéine 4-hydroxybutyrate déshydrogénase.

55. Procédé selon la revendication 38, dans lequel la cellule est en outre apte à produire une protéine succinate-semialdéhyde déshydrogénase et une protéine 4-hydroxybutyrate déshydrogénase.

56. Procédé selon la revendication 38, dans lequel la cellule est en outre apte à produire une protéine 2-méthylcitrate synthase, une protéine 2-méthylcitrate déshydratase, une protéine 2-méthylisocitrate déshydratase, une protéine 2-méthylisocitrate lyase, une protéine succinate:acétyl-CoA transférase, une protéine succinate-semialdéhyde déshydrogénase et une protéine 4-hydroxy-butyrate déshydrogénase.

57. Procédé selon la revendication 38, dans lequel le polyester est un homopolyester.

58. Procédé selon la revendication 57, dans lequel l'homopolyester est un poly(acide 4-hydroxybutyrique).

59. Procédé selon la revendication 57, dans lequel l'homopolyester est un poly(acide 3-hydroxybutyrique).

60. Procédé selon la revendication 38, dans lequel le polyester est un copolyester.

61. Procédé selon la revendication 60, dans lequel le copolyester est un poly(acide 3-hydroxybutyrique-co-acide 4-hydroxybutyrique).

62. Segment d'acide nucléique isolé, vecteur recombinant ou cellule selon l'une quelconque des revendications 1 à 25, comprenant en outre un gène recombinant codant de la β-cétothiolase.
